(19) Europäisches Patentamt
European Patent Office
Office européen des brevets

(11) **EP 3 986 371 B1**

(12) # EUROPÄISCHE PATENTSCHRIFT

(45) Veröffentlichungstag und Bekanntmachung des Hinweises auf die Patenterteilung:
**20.08.2025 Patentblatt 2025/34**

(21) Anmeldenummer: **20720433.0**

(22) Anmeldetag: **20.04.2020**

(51) Internationale Patentklassifikation (IPC):
***A61K 8/86*** *(2006.01)* ***A61K 8/898*** *(2006.01)*
***A61Q 5/06*** *(2006.01)*

(52) Gemeinsame Patentklassifikation (CPC):
**A61K 8/898; A61K 8/86; A61Q 5/065**

(86) Internationale Anmeldenummer:
**PCT/EP2020/060969**

(87) Internationale Veröffentlichungsnummer:
**WO 2020/254008 (24.12.2020 Gazette 2020/52)**

(54) **MITTEL ZUM FÄRBEN VON KERATINISCHEM MATERIAL MIT AMINOSILIKON, FARBGEBENDER VERBINDUNG, KONSERVIERUNGSMITTEL UND ETHOXYLIERTEM FETTALKOHOL**

PRODUCT FOR DYEING KERATINOUS MATERIAL, CONTAINING AMINOSILICONE, A CHROMOPHORIC COMPOUND, PRESERVATIVE AND ETHOXYLATED FATTY ALCOHOL

PRODUIT DE COLORATION D'UNE MATIÈRE KÉRATINIQUE COMPRENANT UNE AMINOSILICONE, UN COMPOSÉ COLORANT, UN CONSERVATEUR ET UN ALCOOL GRAS ÉTHOXYLÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.06.2019 DE 102019208905**

(43) Veröffentlichungstag der Anmeldung:
**27.04.2022 Patentblatt 2022/17**

(73) Patentinhaber: **Henkel AG & Co. KGaA**
**40589 Düsseldorf (DE)**

(72) Erfinder:
- **KRUCK, Constanze**
  **41515 Grevenbroich (DE)**
- **HILBIG, Sandra**
  **44894 Bochum (DE)**
- **BAUMANN, Sofie**
  **42859 Remscheid (DE)**
- **KESSLER-BECKER, Daniela**
  **51371 Leverkusen (DE)**

(56) Entgegenhaltungen:
**WO-A1-2020/126137** **DE-A1- 102008 004 974**
**US-A1- 2013 149 358** **US-A1- 2014 298 598**
**US-A1- 2016 235 657**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 986 371 B1

## Beschreibung

"Mittel zum Färben von keratinischem Material mit Aminosilikon, farbgebender Verbindung, Konservierungsmittel und ethoxyliertem Fettalkohol"

Gegenstand der vorliegenden Anmeldung ist ein Mittel zum Färben von keratinischem Material, insbesondere menschlichen Haaren, welches mindestens ein aminofunktionalisiertes Silikonpolymer (a1), mindestens eine farbgebende Verbindung (a2), mindestens ein Konservierungsmittel (a3) sowie mindestens ein nichtionisches Tensid aus der Gruppe der Anlagerungsprodukte von Ethylenoxid an $C_8$-$C_{24}$-Fettalkohole (a4) enthält.

Ein zweiter Gegenstand dieser Anmeldung ist ein Verfahren zum Färben von keratinischem Material, insbesondere menschlichen Haaren, wobei ein Mittel des ersten Erfindungsgegenstands auf dem keratinischen Material angewendet, einwirken gelassen und danach wieder mit Wasser ausgewaschen wird.

Die Veränderung von Form und Farbe von keratinischem Material, insbesondere von menschlichen Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit tensidhaltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus. Eine nach wie vor bestehende Herausforderung ist daher die Suche nach alternativen, leistungsstarken Färbeverfahren. Vor allem die Waschechtheit von Färbemitteln, die auf dem Einsatz von Pigmenten basieren, ist noch stark verbesserungswürdig.

Es war die Aufgabe der vorliegenden Erfindung, ein Färbesystem bereitzustellen, das mit der oxidativen Färbung vergleichbare Farbintensitäten besitzt. Hierbei sollte jedoch auf den Einsatz der sonst zu diesem Zweck üblicherweise eingesetzten Oxidationsfarbstoffvorprodukte verzichtet werden. Es wurde nach einer Technologie gesucht, die es ermöglicht, die aus dem Stand der Technik bekannten farbgebenden Verbindungen (wie insbesondere Pigmente) in extrem dauerhafter Weise auf den Haaren zu fixieren. Bei Anwendung der Mittel in einem Färbeverfahren sollten besonders intensive Färbeergebnisse mit guten Echtheitseigenschaften erzielt werden. Die Mittel sollten vor allem eine verbesserte Waschechtheit besitzen.

Überraschenderweise hat sich nun herausgestellt, dass die vorgenannte Aufgabe hervorragend gelöst werden kann, wenn keratinische Materialien, insbesondere Haare, mit einem Mittel gefärbt werden, welches mindestens ein aminofunktionalisiertes Silikonpolymer (a1), mindestens eine farbgebende Verbindung (a2), mindestens ein Konservierungsmittel (a3) und mindestens ein nichtionisches Tensid aus der Gruppe der ethoxylierten C8-C24-Fettalkohole (a4) enthält.

Ein erster Gegenstand der vorliegenden Erfindung ist ein Mittel zum Färben von keratinischem Material, insbesondere menschlichen Haaren, enthaltend

(a1) mindestens ein aminofunktionalisiertes Silikonpolymer, das Struktureinheiten der Formel (Si-I) und (Si-II) umfasst, und

(a2) mindestens eine farbgebende Verbindung, und

(a3) mindestens ein Konservierungsmittel aus der Gruppe aus 2-Phenoxyethanol, 4- Hydroxybenzoesäuremethylester, 4-Hydroxybenzoesäuerpropylester, Benzylalkohol, 1-Phenoxy-propan-2-ol, Isopropanol, Ethanol, Zinkpyrithion, Benzoesäure, Salicylsäure, Sorbinsäure, Ameisensäure, Propionsäure, 2-Hydroxydiphenyl, 4- Hydroxybenzoesäure, Dehydracetsäure, Dibromhexamidin, 10-Undecylensäure, Hexetidinum, Trichlorcarban, Triclosanum, 4-Chlor-3,4-dimethylphenol, Imidazolidinylharnstoff, Hexamethylentetramin, 1-(4-Chlorphenoxy)-1-(imidazol-1-yl)-3,3-dimethyl-2- butanon, 1,3-Bis-(hydroxymethyl)-5,5-dimethyl-2,4-imidazolidindion , 1-Hydroxy-4-methyl-6-(2,4,4-trimethylpentyl)-2-pyridon, Bromchlorophen, 3-Methyl-4-(1-methylethyl)phenol, 5- Chlor-2-methyl-3(2H)-isothiazolon, 2-Benzyl-4-chlorphenol, 2-Chloracetamid, Chlorhexidin, 4,4-Dimethyl-1,3-oxazolidin, Hexamidinum, 5-Ethyl-1-aza-3,7-dioxabicyclo-[3.3.0]-octan, Chlorphenesin, Natriumhydroxymethyl-aminoacetat, Benzylhemiformal, 3-Iod-2-propinyl-butylcarbamat, Methylisothiazolinon und Ethyl Lauroyl Arginat, und
(a4) mindestens ein nichtionisches Tensid aus der Gruppe der Anlagerungsprodukte von Ethylenoxid an $C_8$-$C_{24}$-Fettalkohole.

Im Zuge der zu dieser Erfindung durchgeführten Arbeiten hat sich überraschenderweise gezeigt, dass der Einsatz eines Konservierungsmittels (a3) und eines speziellen nichtionischen Tensids (a4) in einem Mittel, welches ein Aminosilikon (a1) sowie eine farbgebende Verbindung (a2) enthält, zu einer Verbesserung der Waschechtheit führt, wenn dieses Mittel in einem Färbeverfahren auf dem keratinischen Material, insbesondere auf menschlichen Haaren, angewendet wird.

<u>keratinisches Material</u>

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.
Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

<u>Mittel zur Färbung</u>

Der Begriff "Mittel zur Färbung" wird im Rahmen dieser Erfindung für eine durch Einsatz von farbgebenden Verbindungen, insbesondere Pigmenten, hervorgerufene Farbgebung des Keratinmaterials, insbesondere des Haares, verwendet. Bei dieser Färbung lagern sich die Pigmente als farbgebende Verbindungen in einem besonders homogenen, gleichmäßigen und glatten Film an der Oberfläche des Keratinmaterials ab.

<u>aminofunktionalisierte Silikonpolymere (a1)</u>

Als ersten erfindungswesentlichen Inhaltsstoff (a1) enthält das Mittel mindestens ein aminofunktionalisiertes Silikonpolymer. Das aminofunktionalisiertes Silikonpolymer kann alternativ auch als Aminosilikon oder Amodimethicone bezeichnet werden.
Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens 5000 g/mol, welche sich wiederholende organische Einheiten umfassen.
Das maximale Molekulargewicht des Silikonpolymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des Silikonpolymers nicht mehr als $10^7$ g/mol, bevorzugt nicht mehr als $10^6$ g/mol und besonders bevorzugt nicht mehr als $10^5$ g/mol beträgt.
Die Silikonpolymere umfassen viele Si-O-Wiederholungseinheiten, wobei die Si-Atome organische Reste wie beispielsweise Alkylgruppen oder substituierte Alkylgruppen tragen können. Alternativ wird ein Silikonpolymer daher auch als Polydimethylsiloxan bezeichnet.
In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.
Färbungen mit den allerbesten Waschechtheiten konnten erhalten werden, wenn im erfindungsgemäßen Verfahren mindestens ein Mittel (a) auf dem keratinischen Material appliziert wurde, das mindestens ein aminofunktionalisiertes Silikonpolymer (a1) enthält, das Struktureinheiten der Formel (Si-I) und der Formel (Si-II) umfasst

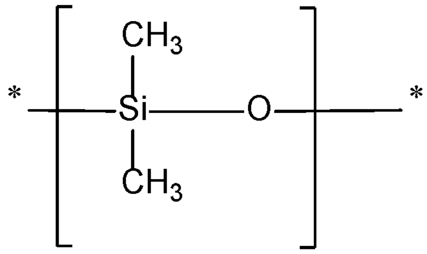
(Si-I)
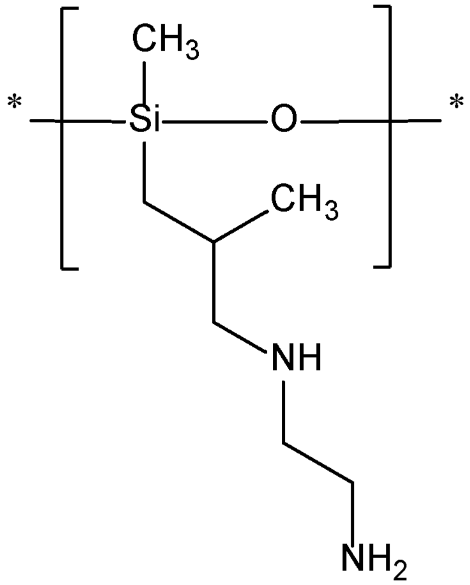
(Si-II).

In einer weiteren explizit ganz besonders bevorzugten Ausführungsform ist ein erfindungsgemäßes Verfahren dadurch gekennzeichnet, dass das Mittel (a) mindestens ein aminofunktionalisiertes Silikonpolymer (a1) enthält, das Struktureinheiten der Formel (Si-I) und der Formel (Si-II) umfasst

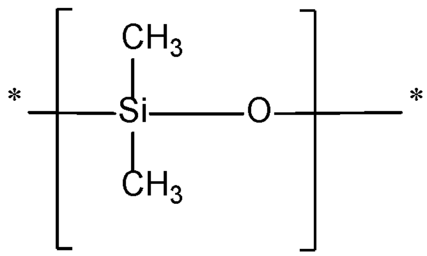
(Si-I)
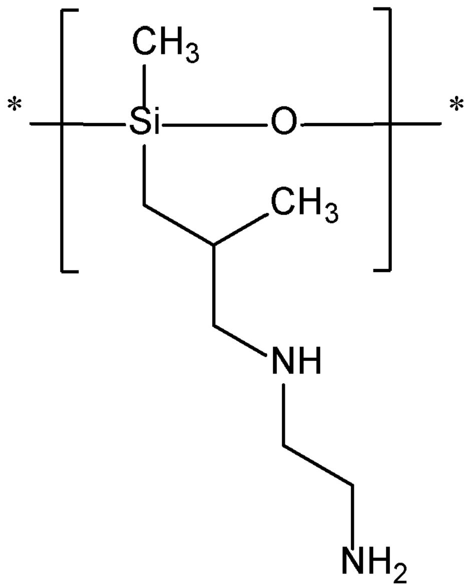
(Si-II).

Ein entsprechendes aminofunkionalisiertes Silikonpolymer mit den Struktureinheiten (Si-I) und (Si-II) ist beispielweise das Handelsprodukt DC 2-8566 bzw. Dowsil 2-8566 Amino Fluid, das von der Firma Dow Chemical Company komerziell vertrieben wird und welches die Benennung "Siloxanes and Silicones, 3-[(2-Aminoethyl)amino]-2-methylpropyl Me, Di-Me-Siloxane" sowie die CAS-Nummer 106842-44-8 trägt.

Das Mittel (a) kann weiterhin auch ein oder mehrere verschiedene aminofunktionalisierte Silikonpolymere enthalten, die durch die Formel (Si-VI)

$$M(R_aQ_bSiO_{(4-a-b)/2})_x(R_cSiO_{(4-c)/2})_yM \qquad \text{(Si-VI)}$$

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel $-R^1HZ$ ist, worin $R^1$ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein

organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3- Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von $R^1$ schließen Methylen, Ethylen, Propylen, Hexamethylen, Decamethylen, - $CH_2CH(CH_3)CH_2$-, Phenylen, Naphthylen, -$CH_2CH_2SCH_2CH_2$-, -$CH_2CH_2OCH_2$-, -$OCH_2CH_2$-, -$OCH_2$ $CH_2CH_2$-, -$CH_2CH(CH_3)C(O)OCH_2$-, -$(CH_2)_3$ $CC(O)OCH_2CH_2$-, -$C_6H$ $_4C_6H_4$-, -$C_6H$ $_4CH_2C_6H_4$-; und -(CH $_2)_3C(O)SCH_2CH_2$- ein.

Z ist ein organischer, aminofunktioneller Rest, enthaltend mindestens eine funktionelle Aminogruppe. Eine mögliche Formel für Z ist $NH(CH_2\ )_zNH_2$, worin z 1 oder mehr ist. Eine andere mögliche Formel für Z ist -$NH(CH_2)_z(CH$ $_2)_{zz}NH$, worin sowohl z als auch zz unabhängig 1 oder mehr sind, wobei diese Struktur Diamino-Ringstrukturen umfaßt, wie Piperazinyl. Z ist am bevorzugtesten ein -$NHCH_2CH$ $_2NH_2$-Rest. Eine andere mögliche Formel für Z ist - $N(CH_2)_z$ $(CH_2)_{zz}NX_2$ oder -$NX_2$, worin jedes X von $X_2$ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -$CH_2CH_2CH_2NHCH_2CH_2NH$ $_2$. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der $R_aQ_b\ SiO_{(4-a-b)/2}$-Einheiten zu den $R_cSiO\ _{(4-c)/2}$-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Siliconkomponenten, die in der Siliconmischung vorhanden sind, verschieden sein.

Im Rahmen einer weiteren bevorzugten Ausführungsform ist ein erfindungsgemäßes Verfahren gekennzeichnet durch die Anwendung eines Mittels (a) auf dem keratinischem Material, wobei das Mittel (a) mindestens ein aminofunktionelles Silikonpolymer (a1) der Formel (Si-VIIa) enthält,

$$(CH_3)_3Si\text{-}[O\text{-}Si(CH_3)_2]_n[OSi(CH_3)]_m\text{-}OSi(CH_3)_3 \qquad \text{(Si-VIIa)},$$

$$|$$

$$CH_2CH(CH_3)CH_2NH(CH_2)_2NH_2$$

worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Unabhängig davon, welche aminofunktionellen Silicone eingesetzt werden, sind erfindungsgemäße Mittel (a) bevorzugt, die ein aminofunktionelles Silikonpolymer enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Es hat sich als besonders vorteilhaft herausgestellt, wenn das erfindungsgemäße Mittel das oder die aminofunktionalisierten Silikonpolymere (a1) in bestimmten Mengenbereichen enhält. Besonders gute Ergebnisse konnten erhalten werden, wenn das Mittel - bezogen auf das Gesamtgewicht des Mittels - in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

Im Rahmen einer weiteren besonders bevorzugten Ausführungsform ist ein erfindungsgemäßes Mittel dadurch gekennzeichnet, dass es - bezogen auf das Gesamtgewicht des Mittels - ein oder mehrere aminofunktionalisierte Silikonpolymere (a1) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

farbgebende Verbindungen (a2)

Als zweiten wesentlichen Bestandteil enthält das erfindungsgemäße Mittel mindestens eine farbgebende Verbindung (a2).

Unter farbgebenden Verbindungen werden im Sinne der Erfindung Substanzen verstanden, die in der Lage sind, dem Keratinmaterial eine Färbung zu verleihen. Besonders gut geeignete farbgebende Verbindungen können ausgewählt werden aus der Gruppe der Pigmente, der direktziehenden Farbstoffe, der photochromen Farbstoffe und der thermochromen Farbstoffe.

Im Rahmen einer weiteren bevorzugten Ausführungsform ist ein erfindungsgemäßes Mittel dadurch gekennzeichnet, dass es mindestens eine farbgebende Verbindung (a2) aus der Gruppe der Pigmente, der direktziehenden Farbstoffe, der photochromen Farbstoffe und der thermochromen Farbstoffe enthält.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Farbpigmente können anorganischen und/oder organischen Ursprungs sein.

In einer bevorzugten Ausführungsform ist ein erfindungsgemäßes Mittel dadurch gekennzeichnet, dass es mindestens eine farbgebende Verbindung (a2) aus der Gruppe der anorganischen und/oder organischen Pigmente enthält.

Bevorzugte Farbpigmente sind ausgewählt aus synthetischen oder natürlichen anorganischen Pigmenten. Anorganische Farbpigmente natürlichen Ursprungs können beispielsweise aus Kreide, Ocker, Umbra, Grünerde, gebranntem Terra di Siena oder Graphit hergestellt werden. Weiterhin können als anorganische Farbpigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Farbpigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Erfindungsgemäß ebenfalls besonders bevorzugte Farbpigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

In einer weiteren bevorzugten Ausführungsform ist ein erfindungsgemäßes Mittel dadurch gekennzeichnet, dass es mindestens eine farbgebende Verbindung (a2) aus der Gruppe der anorganischen Pigmente enthält, das bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist ein erfindungsgemäßes Mittel dadurch gekennzeichnet, dass es (a) mindestens eine farbgebende Verbindung (a2) aus der Gruppe der Pigmente enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Beispiele für besonders geeignete Farbpigmente sind im Handel beispielsweise unter den Handelsbezeichnungen Rona®, Colorona®, Xirona®, Dichrona® und Timiron® von der Firma Merck, Ariabel® und Unipure® von der Firma

Sensient, Prestige® von der Firma Eckart Cosmetic Colors und Sunshine® von der Firma Sunstar erhältlich.

Ganz besonders bevorzugte Farbpigmente mit der Handelsbezeichnung Colorona® sind beispielsweise:

Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)

Weiterhin besonders bevorzugte Farbpigmente mit der Handelsbezeichnung Xirona® sind beispielsweise:

Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.

Zudem sind besonders bevorzugte Farbpigmente mit der Handelsbezeichnung Unipure® beispielsweise:

Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Im Rahmen einer weiteren Ausführungsform kann das erfindungsgemäße Mittel auch ein oder mehrere farbgebende Verbindungen (a2) aus der Gruppe der organischen Pigmente enthalten

Bei den erfindungsgemäßen organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI

47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist ein erfindungsgemäßes Mittel dadurch gekennzeichnet, dass es mindestens eine farbgebende Verbindungen (a2) aus der Gruppe der organischen Pigmente enthält, die bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Aufgrund ihrer ausgezeichneten Licht- und Temperaturbeständigkeit ist die Verwendung der zuvor genannten Pigmente in dem Mittel (a) des erfindungsgemäßen Verfahrens ganz besonders bevorzugt. Weiterhin ist es bevorzugt, wenn die eingesetzten Pigmente eine bestimmte Teilchengröße aufweisen. Es ist daher erfindungsgemäß vorteilhaft, wenn das mindestens eine Pigment eine mittlere Teilchengröße $D_{50}$ von 1,0 bis 50 $\mu$m, vorzugsweise von 5,0 bis 45 $\mu$m, bevorzugt von 10 bis 40 $\mu$m, insbesondere von 14 bis 30 $\mu$m, aufweist. Die mittlere Teilchengröße $D_{50}$ kann beispielsweise unter Verwendung von dynamischer Lichtstreuung (DLS) bestimmt werden.

Die farbgebenden Verbindungen (a2), inbesondere die farbgebenden Verbindungen aus der Gruppe der Pigmente, stellen den zweiten wesentlichen des erfindungsgemäßen Mittels dar und werden bevorzugt in bestimmten Mengenbereichen im Mittel eingesetzt.

Besonders gute Ergebnisse wurden erhalten, wenn das Mittel - bezogen auf das Gesamtgewicht des Mittels - ein odere mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthielt.

In einer weiteren ganz besonders bevorzugten Ausführungsform ist ein erfindungsgemäßes Mittel dadurch gekennzeichnet, dass das Mittel - bezogen auf das Gesamtgewicht des Mittels - ein odere mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

Als farbgebende Verbindungen (a2) können die erfindungsgemäßen Mittel auch einen oder mehrere direktziehende Farbstoffe enthalten. Bei direktziehenden Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1,0 g/L.

Direktziehende Farbstoffe können in anionische, kationische und nichtionische direktziehende Farbstoffe unterteilt werden.

In einer weiteren Ausführungsform ist ein erfindungsgemäßes Verfahren dadurch gekennzeichnet, dass das Mittel (a) mindestens eine farbgebende Verbindung (a2) aus der Gruppe der anionischen, nichtionischen und kationischen direktziehenden Farbstoffe enthält.

Geeignete kationische direktziehende Farbstoffe sind beispielsweise Basic Blue 7, Basic Blue 26, HC Blue 16, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76.

Als nichtionische direktziehende Farbstoffe können beipsielsweise nichtionische Nitro- und Chinonfarbstoffe und neutrale Azofarbstoffe eingesetzt werden. Geeigente nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten

Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung ($-SO_3H$) besitzen. In Abhängigkeit vom pH-Wert liegen die protonierten Formen (-COOH, $-SO_3H$) der Carbonsäure- bzw. Sulfonsäuregruppierungen mit ihren deprotonierten Formen ($-COO^-$, $-SO_3^-$ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Erfindungsgemäße Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1,0 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Im Rahmen einer weiteren Ausführungsform ist ein Verfahren zum Färben von keratinischem Material dadurch gekennzeichnet ist, dass das Mittel (a) mindestens einen anionischen direktziehenden Farbstoff enthält, der ausgewählt ist aus der Gruppe der Nitrophenylendiamine, der Nitroaminophenole, der Azofarbstoffe, der Anthrachinonfarbstoffe, der Triarylmethanfarbstoffe, der Xanthen-Farbstoffe, der Rhodamin-Farbstoffe, der Oxazinfarbstoffen und/oder der Indophenolfarbstoffe, wobei die Farbstoffe aus der vorgenannten Gruppe jeweils mindestens eine Carbonsäuregruppe (-COOH), eine Natriumcarboxylatgruppe (-COONa), eine Kaliumcarboxylatgruppe (-COOK), eine Sulfonsäuregruppe ($-SO_3H$) eine Natriumsulfonatgruppe ($-SO_3Na$) und/oder eine Kaliumsulfonatgruppe ($-SO_3K$) besitzen.

Als geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, Iodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg

bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intensität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1,0 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriuimsalze von Mono- und Sisulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatirumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 GEw.-%.

Acid Red 33 ist das Diantriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1 ,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

In einer weiteren Ausführungsform ist ein erfindungsgemäßes Mittel daher dadurch gekennzeichnet, dass es mindestens einen direktziehenden Farbstoff (a2) enthält, der ausgewählt ist aus der Gruppe aus Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Der oder die direktziehenden Farbstoffe können je nach erwünschter Farbintensität in verschiedenen Mengen im Mittel eingesetzt werden. Gute Ergebnisse konnten erhalten werden, wenn das Mittel - bezogen auf das Gesamtgewicht des Mittels - ein oder mehrere direktziehende Farbstoffe (a2) in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthält.

Weiterhin kann das Mittel als farbgebende Verbindung (a2) auch mindestens einen photochromen oder thermochromen Farbstoff enthalten.

Photochrome Farbstoffe sind Farbstoffe, die auf Bestrahlung mit UV-Licht (Sonnenlicht oder Schwarzlicht) mit einer reversiblen Farbtonänderung reagieren. Dabei verändert das UV-Licht die chemische Struktur der Farbstoffe und damit ihr Absorptionsverhalten (Photochromie).

Thermochrome Farbstoffe sind Farbstoffe, die auf Temperaturänderungen mit einer reversiblen Farbtonänderung reagieren. Dabei verändert die Temperaturänderung die chemische Struktur der Farbstoffe und damit ihr Absorptionsverhalten (Thermochromie).

Das Mittel kann - bezogen auf das Gesamtgewicht des Mittels - ein oder mehrere photochrome Farbstoffe (a2) in einer Gesamtmenge von 0,01 bis 10,0 Gew.-%, bevorzugt von 0,1 bis 8,0 Gew.-%, weiter bevorzugt von 0,2 bis 6,0 Gew.-% und ganz besonders bevorzugt von 0,5 bis 4,5 Gew.-% enthalten

<u>Konservierungsmittel (a3)</u>

Als dritten wesentlichen Inhaltsstoff enthalten die erfindungsgemäßen Mittel mindestens einen Konservierungsstoff (a3),aus der Gruppe aus 2-Phenoxyethanol, 4- Hydroxybenzoesäuremethylester, 4-Hydroxybenzoesäuerpropylester, Benzylalkohol, 1-Phenoxy-propan-2-ol, Isopropanol, Ethanol, Zinkpyrithion, Benzoesäure, Salicylsäure, Sorbinsäure, Ameisensäure, Propionsäure, 2-Hydroxydiphenyl, 4- Hydroxybenzoesäure, Dehydracetsäure, Dibromhexamidin, 10-Undecylensäure, Hexetidinum, Trichlorcarban, Triclosanum, 4-Chlor-3,4-dimethylphenol, Imidazolidinyl- harnstoff, Hexamethylentetramin, 1-(4-Chlorphenoxy)-1-(imidazol-1-yl)-3,3-dimethyl-2- butanon, 1,3-Bis-(hydroxymethyl)-5,5-dimethyl-2,4-imidazolidindion , 1-Hydroxy-4-methyl- 6-(2,4,4-trimethylpentyl)-2-pyridon, Bromchlorophen, 3-Methyl-4-(1-methylethyl)phenol, 5- Chlor-2-methyl-3(2H)-isothiazolon, 2-Benzyl-4-chlorphenol, 2-Chloracetamid, Chlorhexidin, 4,4-Dimethyl-1,3-oxazolidin, Hexamidinum, 5-Ethyl-1-aza-3,7- dioxabicyclo-[3.3.0]-octan, Chlorphenesin, Natriumhydroxymethyl-aminoacetat, Benzylhemiformal, 3-Iod-2-propinyl-butylcarbamat, Methylisothiazolinon und Ethyl Lauroyl Arginat. Es hat sich überraschenderweise herausgestellt, dass die normalerweise zur Konservierung eingesetzten Stoffe in den erfindungsgemäßen Mitteln zu Färberesultaten mit verbesserter Waschechehteiten führen.

Der Begriff Konservierungsmittel ist Sammelbegriff für solche Stoffe, die kosmetischen Formulierungen zugesetzt werden, um deren Haltbarkeit gegenüber der Einwirkung von Mikroorganismen, Insekten und anderen Kleinlebewesen zu verlängern.

Ein Konservierungmittel (a3) im Sinne der vorliegenden Erfindung ist eine Substanz, dessen Einsatz im erfindungsgemäßen Mittel dazu führt, dass diese den Konservierungsmittelbelastungstest gemäß Ph. Eur. (Europäisches Arzneibuch), 6. Ausgabe, 5.3.1 besteht. Der Konservierungsmittelbelastungstest wird wie folgt ausgeführt.

30 g des zu testenden erfindungsgemäßen Mittels werden jeweils mit $10^5$ koloniebildenden Einheiten (KBE) pro 1 g Zusammensetzung der folgenden Testmikroorganismen geimpft: Pseudomonas aeruginosa (Bakterium), Staphylococcus aureus (Bakterium), Candida albicans (Pilz), Aspergillus brasiliensis (Pilz). Nach Zugabe des jeweiligen Mikroorganismus wird die Probe durch Rühren mittels eines Glasstabes homogenisiert und anschließend bei 20 bis 25 °C im Dunkeln gelagert. Nach 7, 14, 21 oder 28-tägiger Lagerung der geimpften Zusammensetzungen wurde jeweils 1 g der jeweiligen Probe entnommen und die darin enthaltenen KBE bestimmt. Ziel der Konservierung ist eine Reduktion der KBE auf einen Wert unterhalb der Nachweisgrenze (für jeden Typ zugesetzter Mikroorganismen). Der Konservierungsmitteltest gilt als bestanden, wenn die KBE nach spätestens 28 Tagen unterhalb der Nachweisgrenze liegt.

Eine besonders starke Verbesserung der Waschechtheit konnte beobachtet werden, wenn das Konservierungsmittel (a3) aus der Gruppe ausgewählt wurde, die gebildet wird aus 2-Phenoxyethanol, 4-Hydroxybenzoesäuremethylester, 4-Hydroxybenzoesäuerpropylester, Benzylalkohol, 1-Phenoxy-propan-2-ol, Isopropanol, Ethanol, Zinkpyrithion, Benzoesäure, Salicylsäure, Sorbinsäure, Ameisensäure, Propionsäure, 2-Hydroxydiphenyl, 4-Hydroxybenzoesäure, Dehydracetsäure, Dibromhexamidin, 10-Undecylensäure, Hexetidinum, Trichlorcarban, Triclosanum, 4-Chlor-3,4-dimethylphenol, Imidazolidinylharnstoff, Hexamethylentetramin, 1-(4-Chlorphenoxy)-1-(imidazol-1-yl)-3,3-dimethyl-2-butanon, 1,3-Bis-(hydroxymethyl)-5,5-dimethyl-2,4-imidazolidindion , 1-Hydroxy-4-methyl-6-(2,4,4-trimethylpentyl)-2-pyridon, Bromchlorophen, 3-Methyl-4-(1-methylethyl)phenol, 5-Chlor-2-methyl-3(2H)-isothiazolon, 2-Benzyl-4-chlorphenol, 2-Chloracetamid, Chlorhexidin, 4,4-Dimethyl-1,3-oxazolidin, 1-Phenoxy-propan-2-ol, Hexamidinum, 5-Ethyl-1-aza-3,7-dioxabicyclo-[3.3.0]-octan, Chlorphenesin, Natriumhydroxymethyl-aminoacetat, Benzylhemiformal, 3-Iod-2-propinyl-butylcarbamat, Methylisothiazolinon und Ethyl Lauroyl Arginat enthält.

Im Rahmen einer weiteren Ausführungsform ist ein ganz besonders bevorzugtes Mittel dadurch gekennzeichnet, dass es mindestens ein Konservierungsmittel (a3) aus der Gruppe aus 2-Phenoxyethanol, 4-Hydroxybenzoesäuremethylester, 4-Hydroxybenzoesäuerpropylester, Benzylalkohol, 1-Phenoxy-propan-2-ol, Isopropanol, Ethanol, Zinkpyrithion, Benzoesäure, Salicylsäure, Sorbinsäure, Ameisensäure, Propionsäure, 2-Hydroxydiphenyl, 4-Hydroxybenzoesäure, Dehydracetsäure, Dibromhexamidin, 10-Undecylensäure, Hexetidinum, Trichlorcarban, Triclosanum, 4-Chlor-3,4-dimethylphenol, Imidazolidinylharnstoff, Hexamethylentetramin, 1-(4-Chlorphenoxy)-1-(imidazol-1-yl)-3,3-dimethyl-2-butanon, 1,3-Bis-(hydroxymethyl)-5,5-dimethyl-2,4-imidazolidindion , 1-Hydroxy-4-methyl-6-(2,4,4-trimethylpentyl)-2-pyridon, Bromchlorophen, 3-Methyl-4-(1-methylethyl)phenol, 5-Chlor-2-methyl-3(2H)-isothiazolon, 2-Benzyl-4-chlorphenol, 2-Chloracetamid, Chlorhexidin, 4,4-Dimethyl-1,3-oxazolidin, 1-Phenoxy-propan-2-ol, Hexamidinum, 5-Ethyl-1-aza-3,7-dioxabicyclo-[3.3.0]-octan, Chlorphenesin, Natriumhydroxymethyl-aminoacetat, Benzylhemiformal, 3-Iod-2-propinyl-butylcarbamat, Methylisothiazolinon und Ethyl Lauroyl Arginat enthält.

2-Phenoxyethanol ist ein Ether des Phenols mit Ethylenglycol mit der Summenformel $C_8H_{10}O_2$. 2-Phenoxyethanol besitzt die CAS-Nummer 122-99-6.

4-Hydroxybenzoesäuremethylester oder auch para-Hydroxybenzoesäuremethylester (ist der Methylester der aromatischen Carbonsäure 4-Hydroxybenzoesäure und gehört zu den Parabenen. 4-Hydroxybenzoesäuremethylester besitzt die CAS-Nummer 99-76-3.

4-Hydroxybenzoesäuerpropylester oder auch para-Hydroxybenzoesäurepropylester der Propylester der aromatischen Carbonsäure 4-Hydroxybenzoesäure und gehört zu den Parabenen. 4-Hydroxybenzoesäuerpropylester trägt die CAS-Nummer 94-13-3.

Benzylalkohol wird alternativ auch als Phenylmethanol bezeichnet und besitzt die CAS-Nummer 100-51-6.

Alternativnamen für 1-Phenoxy-propan-2-ol sind 1-Phenoxy-2-propanol und Phenoxyisopropanol. 1-Phenoxy-propan-2-ol trägt die CAS-Nummer 770-35-4.

Isopropanol wird auch als 2-Propanol bezeichnet und trägt die CAS-Nummer 67-63-0.

Ethanol besitzt die CAS-Nummer 64-17-5.

Zinkpyrithion wird alternativ auch als Zink-bis[2-pyridinthiolat]-*N,N'*-dioxid, als 2-Pyridinthiol-1-oxid, Zinksalz oder als Zink-2-mercaptopyridin-*N*-oxid bezeichnet. Die CAS-Nummer von Zinkpyrithion ist 13463-41-7.

Benzoesäure trägt die CAS-Nummer 65-85-0.

Salicylsäure wird alternativ auch als 2-Hydroxybenzoesäure bezeichnet und trägt die CAS-Nummer 69-72-7.

Sorbinsäure trägt den Alternativnamen 2,4-Hexadiensäure ((2*E*,4*E*)-Hexa-2,4-diensäure) und besitzt die CAS-Nummer 110-44-1. Auch die Salze der Sorbinsäure, insbesondere das Natriumsalz und das Kaliumsalz, sind von der Erfindung mit umfasst.

Ameisensäure wird alterantiv auch als Methansäure bezeichnet und trägt die CAS-Nummer 64-18-6.

Propionsäure wird alternativ auch als Propansäure bezeichnet und trägt die CAS-Nummer 79-09-4.

2-Hydroxydiphenyl wird alternativ auch als Biphenyl-2-ol oder 2-Hydroxybiphenyl oder Orthophenylphenol bezeichnet. 2-Hydroxydiphenyl trägt die CAS-Nummer 90-43-7.

4-Hydroxybenzoesäure wird alternativ auch als p-Salicylsäure, p-Hydroxybenzoesäure bezeichnet und besitzt die CAS-Nummer 99-96-7.

Dehydracetsäure besitzt den Alternativnamen 3-Acetyl-6-methyl-2,4(3H)-pyrandion, trägt die CAS-Nummer 520-45-6 und hat die Struktur (K1). Auch die tautomeren Formen der Dehydracetsäure sind von der Erfindung mit umfasst.

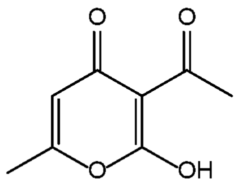

(K1)

Dibromhexamidin wird alternativ auch als 1,6-Bis(4-amidino-2-bromphenoxy)-n-hexan oder als ,4'-(Hexan-1,6-diyl)-bis-(3-brombenzamidin) bezeichnet und besitzt die CAS-Nummer 93856-82-7. Dibromhexamidin hat die Struktur (K2).

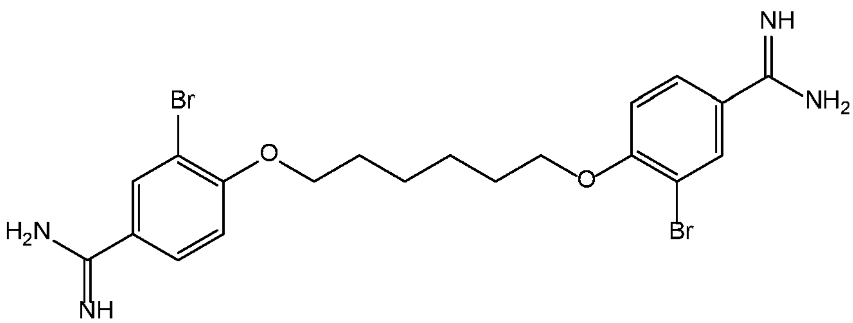

(K2)

10-Undecylensäure trägt die Alternativnamen Undec-10-ensäure oder 10-Undecensäure und hat die CAS-Nummer 112-38-9. 10-Undecylensäure hat die Struktur der Formel (K3). Auch die Salze der 10-Undecylensäure, insbesondere das Natriumsalz und das Kaliumsalz, sind von der Erfindung mit umfasst.

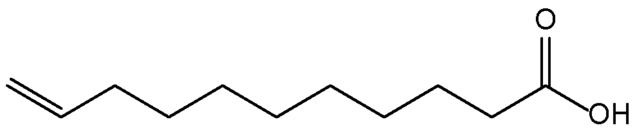

(K3)

Hexetidinum wird alternativ auch als Hexetidin oder 1,3-Bis(2-ethylhexyl)-5-methylhexahydropyrimidin-5-amin bezeichnet. Hexetidinum bzw. Hexetidin besitzt die CAS-Nummer 141-94-6. Hexetidinum bzw. Hexetidin hat die Struktur der Formel (K4).

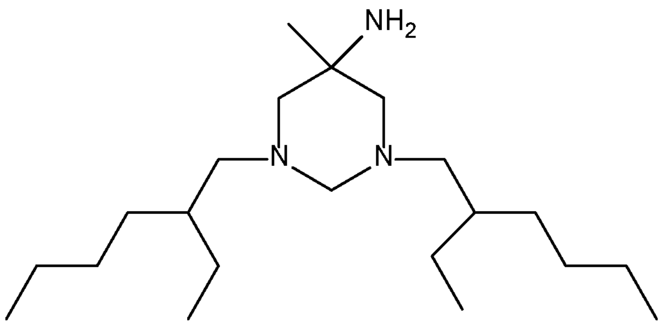

(K4)

Triclocarban trägt auch die Alternativnamen 3,4,4'-Trichlorcarbanilid oder 3-(4-Chlorphenyl)-1-(3,4-dichlorphenyl)harnstoff und besitzt die CAS-Nummer 101-20-2. Triclocarban hat die Struktur der Formel (K5).

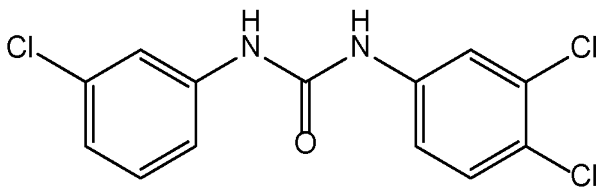

(K5)

Triclosanum oder Triclosan wird alternativ auch als 5-Chlor-2-(2,4-dichlorphenoxy)-phenol bezeichnet. Triclosanum bzw. Trichlosan trägt die CAS-Nummer 3380-34-5. Triclosanum bzw. Triclosan hat die Struktur der Formel (K6).

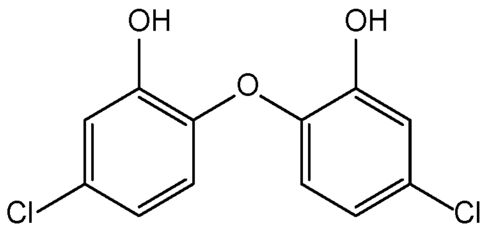

(K6)

4-Chlor-3,4-dimethylphenol wird auch als Chlorxylenol bezeichnet und besitzt die CAS-Nummer 88-04-0.

Imidazolidinylharnstoff wird alternativ auch als *N,N'*-Methylenebis[*N'*-(3-hydroxymethyl-2,5-dioxo-4-imidazolidinyl)urea] bezeichnet. Imidazolidinylharnstoff trägt die CAS-Nummer 39236- 46- 9 und hat die Struktur der Formel (K7).

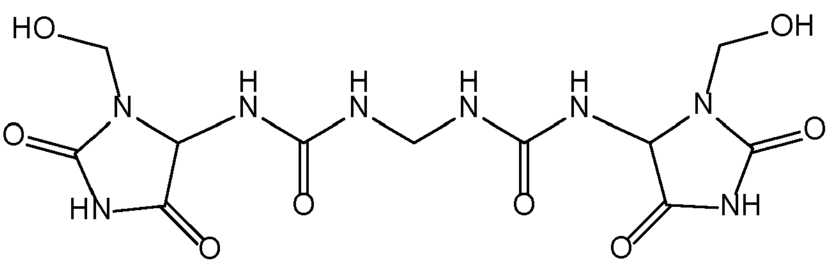

(K7).

Hexamethylentetramin wird auch als Urotropin oder 1,3,5,7-Tetraazaadamantan bezeichnet. Hexamethylentetramin hat die CAS-Nummer 100-97-0.

1-(4-Chlorphenoxy)-1-(imidazol-1-yl)-3,3-dimethyl-2-butanon wird alternativ auch als Climbazol bezeichnet, hat die CAS-Nummer 38083-17-9 und eine Struktur der Formel (K8). Die Struktur K8 umfasst zwei enantiomere Formen. Beide Enantiomere und auch das Gemisch beider Enantiomeren sind von der Erfindung mit umfasst.

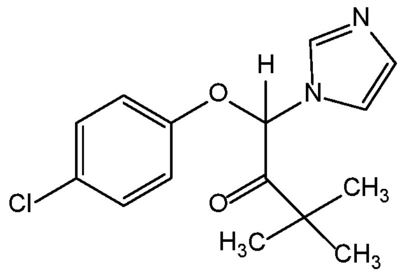

(K8).

1,3-Bis-(hydroxymethyl)-5,5-dimethyl-2,4-imidazolidindion wird auch als DMDM Hydantoin bezeichnet, hat die CAS-Nummer 6440-58-0 und besitzt diie Struktur der Formel (K9).

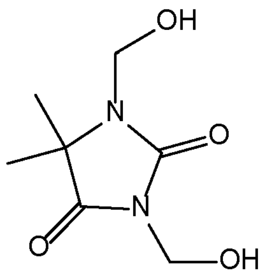

(K9).

1-Hydroxy-4-methyl-6-(2,4,4-trimethylpentyl)-2-pyridon besitzt die Alternativnamen 1-Hydroxy-4-methyl-6-(2,4,4-trimethylpentyl)pyridin-2(1H)-on, Octopirox und Piroctone Olamin und hat die CAS-Nummer 68890-66-4. Besonders bevorzugt wird dieses Konservierungsmittel in Form seines 1:1 Adduktes mit 2-Aminoethanol einsetzt. 1-Hydroxy-4-methyl-6-(2,4,4-trimethylpentyl)-2-pyridon (in Form seines Ethanolaminadduktes) hat die Struktur der Formel (K10).

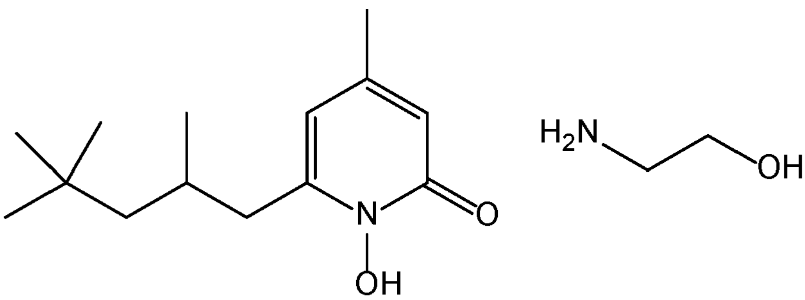

(K10)

Bromchlorophen trägt den Alternativinamen 2,2'-Methylenbis-(6-brom-4-chlorphenol) und hat die CAS-Nummer 15435-29-7. Bromchlorophen besitzt die Struktur der Formel (K11).

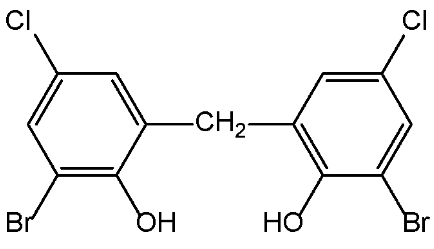

(K11)

3-Methyl-4-(1-methylethyl)phenol trägt die Alternativnamen o-Cymen-5-ol, p-Thymol, Biosol und 1-Hydroxy-3-methyl-4-isopropylbenzene und hat die CAS-Nummer 3228-02-2.

5-Chlor-2-methyl-3(2H)-isothiazolon wird alternativ auch als 5-Chlor-2-methyl-4-isothiazolin-3-on oder Chlormethylisothiazolon bezeichnet, hat die CAS-Nummer 26172-55-4 und besitzt die Struktur der Formel (K12).

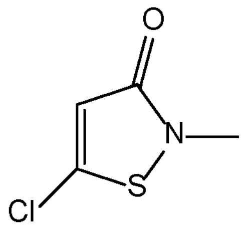

(K12)

2-Benzyl-4-chlorphenol wird alternativ auch als Chlorophenum oder Chlorophen bezeichnet und hat die CAS-Nummer 120-32-1.

2-Chloracetamid trägt den Alternativnamen Chloressigsäureamid und hat die CAS-Nummer 79-07-2.

Chlorhexidin wird alternativ auch als 1,1'-Hexamethylen-bis[5-(4-chlorphenyl)biguanid] bezeichnet und hat die CAS-Nummer 55-56-1. Chlorhexidin hat die Struktur der Formel (K13).

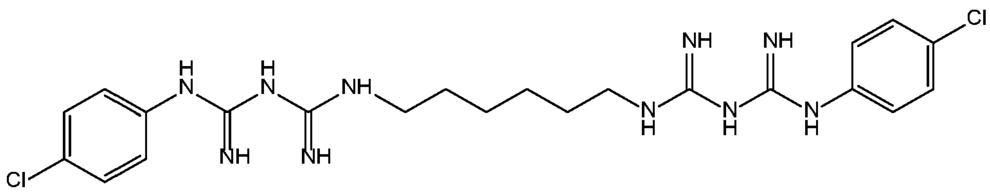

(K13)

4,4-Dimethyl-1,3-oxazolidin hat die CAS-Nummer 51200-87-4.

1-Phenoxy-propan-2-ol wird alternativ auch als Phenyl-β-hydroxypropyl ether, 1-Phenoxy-2-propanol, Phenoxyisopropanol, Propylene phenoxetol, 2-Phenoxy-1-methylethanol oder Propylene glycol 1-phenyl ether bezeichnet und hat die CAS-Nummer 770-35-4.

Hexamidinum wird alternativ auch als Hexamidin oder 1,6-Bis(4-amidinophenoxy)-n-hexan oder 4,4'-[Hexane-1,6-diylbis(oxy)]dibenzenecarboximidamid bezeichnet und hat die CAS-Nummer 3811-75-4.

Hexamidin besitzt die Struktur der Formel (K14).

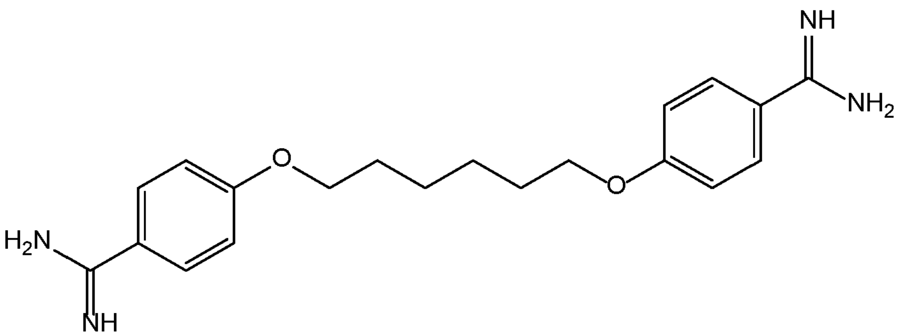

(K14)

5-Ethyl-1-aza-3,7-dioxabicyclo-[3.3.0]-octan wird alternativ auch als 5-Ethyl-3,7-dioxa-1-azabicyclo-[3.3.0]octan oder Dihydro-7a-ethyloxazolo[3,4-*c*]oxazol bezeichnet, hat die CAS-Nummer 7747-35-5 und besitzt die Struktur der Formel (K15)

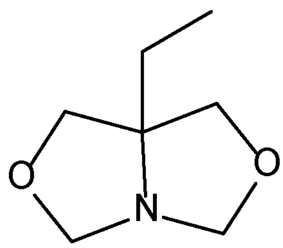

(K15)

Chlorphenesin wird alternativ auch als (3-(4-chlorophenoxy)-2-hydroxypropyl)carbamat bezeichnet und hat die

CAS-Nummer 886-74-8. Chlorphenesin besitzt die Struktur der Formel (K16).

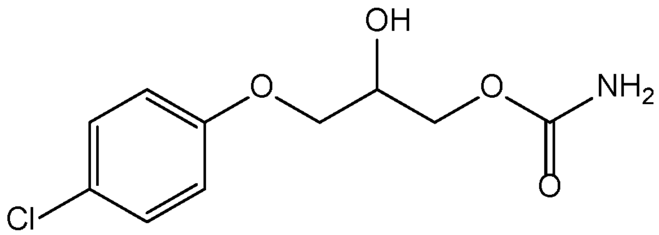

(K16)

Natriumhydroxymethyl-aminoacetat wird alternativ auch als Natrium-N-(hydroxymethyl)glycinat oder Natrium-N-(hydroxymethyl)glycinat bezeichnet, hat die CAS-Nummer 70161-44-3 und besitzt die Struktur der Formel (K17)

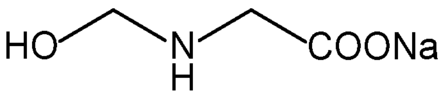

(K17).

Benzylhemiformal wird alteranativ auch als (Benzyloxy)methanol bezeichnet und hat die CAS-Nummer 14548-60-8.

3-Iod-2-propinyl-butylcarbamat wird alternativ auch als 3-Iodpropargyl-*N*-butylcarbamat oder Biodocarb bezeichnet und hat die CAS-Nummer 55406-53-6. 3-Iod-2-propinyl-butylcarbamat besitzt die Struktur der Formel (K18).

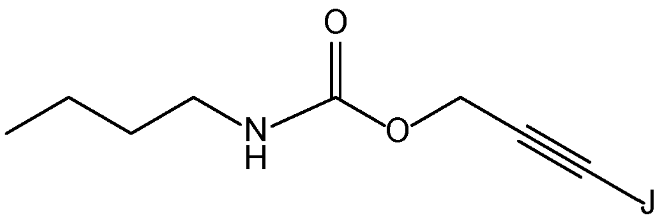

(K18)

Methylisothiazolinon wird alternativ auch als 2-Methyl-2*H*-isothiazol-3-on bezeichnet und hat die CAS-Nummer 2682-20-4

Ethyl Lauroyl Arginat wird alternativ auch als Ethyl-N$\alpha$-dodecanoyl-L-arginate hydrochloride oder Monohydrochloride of L-arginine oder Na-lauroyl-ethylester bezeichnet und hat die CAS-Nummer 60372-77-2. Ethyl Lauroyl Arginat hat die Struktur der Formel (K19) und kann entweder als freie Verbindung oder aber in Form seines Hydrochloridsalzes eingesetzt werden.

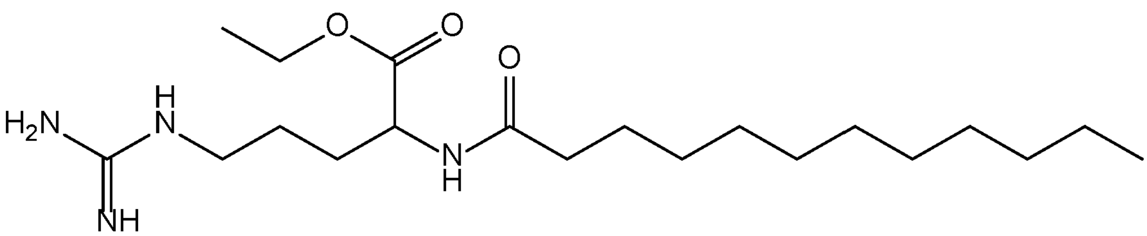

(K19)

Die allerbesten Ergebnisse wurden erhalten, wenn Phenoxyethanol und/oder 4-Hydroxybenzoesäuremethylester als Konservierungsmtitel (a3) eingesetzt wurden, da mit diesen beiden Konservierungsmitteln eine ganz besonders starke Verbesserung der Waschechtheit erzielt werden konnte.

Im Rahmen einer weiteren ganz besonders bevorzugten Ausführungsform ist ein erfindungsgemäßes Mittel dadurch gekennzeichnet, dass es (a3) 2-Phenoxyethanol und/oder 4-Hydroxybenzoesäuremethylester enthält.

Im Rahmen einer weiteren ganz besonders bevorzugten Ausführungsform ist ein erfindungsgemäßes Mittel dadurch gekennzeichnet, dass es (a3) 2-Phenoxyethanol enthält.

Im Rahmen einer weiteren ganz besonders bevorzugten Ausführungsform ist ein erfindungsgemäßes Mittel dadurch gekennzeichnet, dass es (a3) 4-Hydroxybenzoesäuremethylester enthält.

Im Rahmen einer weiteren ganz besonders bevorzugten Ausführungsform ist ein erfindungsgemäßes Mittel dadurch gekennzeichnet, dass es (a3) 2-Phenoxyethanol und 4-Hydroxybenzoesäuremethylester enthält.

Auch die Konservierungsmittel (a3) werden besonders bevorzugt in bestimmten Mengenbereichen im erfindungsgemäßen Mittel eingesetzt.

Besonders gute Ergebnisse wurden erhateln wenn das Mittel - bezogen auf das Gesamtgewicht des Mittels - ein oder mehere Konservierungsmittel (a3) in einer Gesamtmenge von 0,01 bis 5,0 Gew.-%, bevorzugt 0,1 bis 2,5 Gew.-%, weiter bevorzugt von 0,15 bis 1,0 Gew.-% und ganz besonders bevorzugt von 0,2 bis 0,8 Gew.-% enthielt

Im Rahmen einer weiteren bevorzugten Ausführungsform ist ein erfindungsgemäßes Mittel dadurch gekennzeichnet, dass es - bezogen auf das Gesamtgewicht des Mittels - ein oder mehrere Konservierungsmittel (a3) in einer Gesamtmenge von 0,01 bis 5,0 Gew.-%, bevorzugt 0,1 bis 2,5 Gew.-%, weiter bevorzugt von 0,15 bis 1,0 Gew.-% und ganz besonders bevorzugt von 0,2 bis 0,8 Gew.-% enthält.

Auch 2-Phenoxyethanol wird bevorzugt in bestimmten Mengenbereichen im erfindungsgemäßen Mittel eingesetzt. Es hat sich als ganz besonders vorteilhaft herausgestellt, wenn das Mittel - bezogen au das Gesamtgewicht des Mittels - 0,1 bis 1,5 Gew.-%, bevorzugt 0,2 bis 1,0 Gew.-%, weiter bevorzugt 0,3 bis 0,9 und ganz besonders bevorzugt 0,4 bis 0,8 Gew.-% 2-Phenoxyethanol enthält.

Auch 4-Hydroxybenzoesäuremethylester wird bevorzugt in bestimmten Mengenbereichen im erfindungsgemäßen Mittel eingesetzt. Es hat sich als ganz besonders vorteilhaft herausgestellt, wenn das Mittel - bezogen au das Gesamtgewicht des Mittels - 0,1 bis 1,5 Gew.-%, bevorzugt 0,2 bis 1,0 Gew.-%, weiter bevorzugt 0,3 bis 0,9 und ganz besonders bevorzugt 0,3 bis 0,5 Gew.-% 4-Hydroxybenzoesäuremethylester enthält.

<u>nichtionische Tenside (a4)</u>

Als vierten erfindungswesentlichen Bestandteil (a4) enthält das erfindungsgemäße Mittel mindestens ein nichtionisches Tensid aus der Gruppe der Anlagerungsprodukte von Ethylenoxid an $C_8$-$C_{24}$-Fettalkohole.

Die Anlagerung von Ethylenoxid an $C_2$-$C_{24}$-Fettalkohole führt zur Ausbildung von ethoxylierten $C_8$-$C_{24}$-Fettalkoholen. Als Ethoxy-Gruppe wird in diesem Fall die Struktureinheit $-CH_2-CH_2-O-$bezeichnet, die alternativ auch als Ethylenoxid-Einheit bezeichnet werden kann.

Die Molmenge an Ethylenoxid, die pro Mol Fettalkohol eingesetzt wurde, bezeichnet dabei den Ethoxylierungsgrad.

Unter niedrig ethoxylierten Fettalkohlen werden beispielsweise Fettalkohole verstanden, die mit mindestens 1 und höchstens 25 Ethoxygruppen (EO-Gruppen) ethoxyliert wurden. Als Ethoxy-Gruppe wird in diesem Fall wieder die Struktureinheit $-CH_2-CH_2-O-$ bezeichnet, die alternativ auch als Ethylenoxid-Einheit bezeichnet werden kann.

Unter hoch ethoxylierten Fettalkohlen werden analog C8-C24-Fettalkohole verstanden, die mit mindestens 25 und höchstens 100 Ethoxygruppen ethoxyliert wurden (d.h. jedes mol Fettalkohol wurde mit 30 mol bis 100 mol Ethylenoxid ethoxyliert).

Mit anderen Worten handelt es sich bei dem ersten Erfindungsgegenstand um ein Mittel zum Färben von keratinischem Material, insbesondere menschlichen Haaren, enthaltend

(a1) mindestens ein aminofunktionalisiertes Silikonpolymer, und
(a2) mindestens eine farbgebende Verbindung, und
(a3) mindestens ein Konservierungsmittel und
(a4) mindestens ein nichtionisches Tensid aus der Gruppe der ethoxylierten $C_8$-$C_{24}$-Fettalkohole mit 1 bis 100 Ethoxy-Gruppen.

Bei $C_8$-$C_{24}$-Fettalkoholen handelt es sich erfindungsgemäß um lineare oder verzweigte, gesättigte oder ungesättigte Alkanole mit 8 bis 24 Kohlenstoffatomen. Ungesättigte $C_{16}$-$C_{18}$-Fettalkohole können einfach oder mehrfach ungesättigt sein. Bei einem ungesättigten Fettalkohol kann bzw. können dessen C-C-Doppelbindung(en) die Cis- oder Trans-Konfiguration aufweisen. Es ist erfindungsgemäß ebenfalls möglich, Gemische von Fettalkoholen, die durch gezielte Mischung oder auch durch Gewinnungsverfahren als solche anfallen, einzusetzen. Ein Beispiel ist Cetearylalkohol (1:1-Mischung aus $C_{16}$- und $C_{18}$-Fettalkoholen).

Als geeignete ethoxylierte Fettalkohole (a4) können beispielsweise Ceteareth-2, Steareth-2, Ceteth-2, Oleth-2, Ceteareth-3, Steareth-3, Ceteth-3, Oleth-3, Ceteareth-4, Steareth-4, Ceteth-4, Oleth-4, Ceteareth-5, Steareth-5, Ceteth-5 und/oder Oleth-5, Ceteareth-30, Steareth-30, Ceteth-30, Oleth-30, Ceteareth-50, Steareth-50, Ceteth-50, Oleth-50, Ceteareth-100, Steareth-100, Ceteth-100 und Oleth-100 genannt werden.

Besonders starke Verbessungen der Waschechtheit wurden erhalten, wenn das erfindungsgemäße Mittel mindestens einen ethoxylierten Fettalkohol mit einem Ethoxylierungsgrad von 80 bis 120 enthielt.

In einer weiteren ganz besonders bevorzugten Ausführungsform ist ein erfindungsgemäßes Mittel dadurch gekennzeichnet, dass es dass es mindestens ein nichtionisches Tensid (a4) der Formel (T-I) enthält,

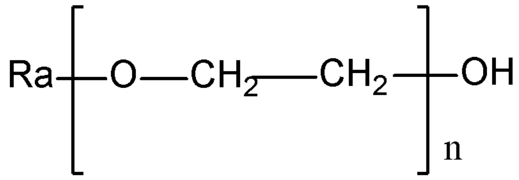

(T-I)

worin

- Ra für eine gesättigte oder ungesättigte, unverzweigte oder verzweigte $C_8$-$C_{24}$-Alkylgruppe, bevorzugt für eine gesättigte, unverzeigte $C_{16}$- bis $C_{18}$- Alkylgruppe, steht und
- n für eine ganze Zahl von 80 bis 120, bevorzugt für eine ganze Zahl von 90 bis 110 und besonders bevorzugt für die Zahl 100, steht.

Ein besonders gut geeignetes nichtionisches Tensid dieses Typs trägt den Handelsnamen Brij S 100 bzw. Brij S 100 PA SG. Hierbei handelt es sich um Stearylalkohol, ethoxyliert mit 100 EO, der von der Firma Croda kommerziell erhältlich ist und die CAS-Nummer 9005-00-9 trägt.

Weiterhin ganz besonders gute Ergebnisse wurden erhalten, wenn ein erfindungsgemäßens Mittel eingesetzt wurde, welche mindestens einen ethoxylierten Fettalkohole mit einem Ethoxylierungsgrad von 10 bis 40 enthielt.

In einer weiteren ganz besonders bevorzugten Ausführungsform ist ein erfindungsgemäßes Mittel dadurch gekennzeichnet, dass es dass es mindestens ein nichtionisches Tensid (a4) der Formel (T-II) enthält,

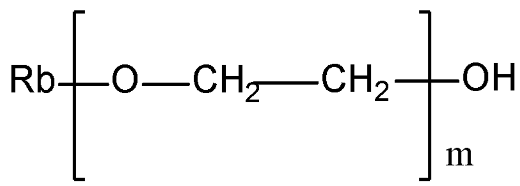

(T-II)

worin

- Rb für eine gesättigte oder ungesättigte, unverzweigte oder verzweigte $C_8$-$C_{24}$-Alkylgruppe, bevorzugt für eine gesättigte, unverzweigte $C_{16}$- bis $C_{18}$- Alkylgruppe, steht und
- m für eine ganze Zahl von 10 bis 40, bevorzugt für eine ganze Zahl von 20 bis 35 und besonders bevorzugt für die Zahl 30, steht.

Bei einem besonders gut geeigneten nichtionischen Tensid dieses Typs handelt es sich um Ceteareth-30. Bei Ceteareth-30 handelt es sich um ein Gemisch aus Cetylalkohol und Stearylalkohol, die jeweils mit 30 Einheiten Ethylenoxid ethoxyliert sind. Das Gemisch aus Cetylalkohol und Stearylalkohol wird als Cetearylalkohol bezeichnet. Ceteareth-30 besitzt die CAS-Nummer 68439-49-6 und ist beispielsweise unter dem Handelsnamen Eumulgin B3 von der Firma BASF käuflich zu erwerben.

Es hat sich als ganz besonders bevorzugt erwiesen, wenn das Mittel sowohl mindestens ein nichtionisches Tensid der Formel (T-I) als auch mindestens ein nichtionisches Tensid der Formel (T-II) enthält.

Es ist ganz besonders bevorzugt, wenn die das Mittel - bezogen auf das Gesamtgewicht des Mittelseinen oder mehrere nichtionische Tenside (a4) aus der Gruppe der Anlagerungsprodukte von Ethylenoxid an $C_8$-$C_{24}$-Fettalkohol in einer Gesamtmenge von 0,1 bis 20,0 Gew.-%, bevorzugt von 0,2 bis 10,0 Gew.-%, weiter bevorzugt von 0,5 bis 8,0 Gew.-%, noch weiter bevorzugt von 1,0 bis 6,0 Gew.-% und ganz besonders bevorzugt von 1,2 bis 4,0 Gew.-% enthält.

Fettbestandteile im Mittel

Als weiteren optionalen Bestandteil kann das erfindungsgemäße Mittel zusätzlich auch noch mindestens einen Fettbestandteil enthalten.

Es hat sich herausgestellt, dass der Einsatz mindestens eines Fettbestandteils dazu führt, dass das Mittel in Form einer Emulsion vorliegt, welche die optimale Viskosität besitzt und sich auch im Hinblick auf die Verbesserung der Farbintensität als vorteilhaft herausgestellt hat.

Bei den Fettbestandteilen handelt es sich um hydrophobe Substanzen, die in Gegenwart von Wasser unter Ausbildung von Mizell-Systemen Emulsionen formen können. Ohne auf diese Theorie festgelegt zu sein, wird vermutet,

dass die $C_1$-$C_6$-Alkoxysilane - entweder in Form ihrer Monomere oder gegebenenfalls in Form ihrer kondensierten Oligomere - in diese hydrophobe Umgebung bzw. in die Mizell-Systeme eingebettet werden, so dass sich die Polarität ihrer Umgebung verändert. Bedingt durch den hydrophoben Charakter der Fettbestandteile wird auch die Umgebung der $C_1$-$C_6$-Alkoxysilane hydrophobiert. Es wird angenommen, dass die zum Film bzw. Coating führende Polymerisationsreaktion der $C_1$-$C_6$-Alkoxy-silane in einem Milieu verringerter Polarität mit reduzierter Geschwindigkeit abläuft.

Unter "Fettbestandteilen" werden im Sinne der Erfindung organische Verbindungen mit einer Löslichkeit in Wasser bei Raumtemperatur (22 °C) und atmosphärischem Druck (760 mmHg) von weniger als 1 Gew.-%, bevorzugt von weniger als 0,1 Gew.-% verstanden. Unter die Definition der Fettbestandteile fallen explizit nur ungeladene (d.h. nichtionische) Verbindungen. Fettbestandteile besitzen mindestens eine gesättigte oder ungesättigte Alkylgruppe mit mindestens 12 C-Atomen. Das Molgewicht der Fettbestandteile liegt bei maximal 5000 g/mol, bevorzugt bei maximal 2500 g/mol und besonders bevorzugt bei maximal 1000 g/mol. Bei den Fettbestandteilen handelt es sich weder um polyoxyalkylierte noch um polyglycerylierte Verbindungen.

Ganz besonders bevorzugt werden die im Mittelenthaltenen Fettbestandteile (a4) ausgewählt aus der Gruppe der $C_{12}$-$C_{30}$-Fettalkohole, der $C_{12}$-$C_{30}$-Fettsäuretriglyceride, der $C_{12}$-$C_{30}$-Fettsäuremonoglyceride, der $C_{12}$-$C_{30}$-Fettsäurediglyceride und/oder der Kohlenwasserstoffe.

Im Rahmen einer weiteren bevorzugten Ausführungsform ist ein erfindungsgemäßes Mittel dadurch gekennzeichnet, dass es einen oder mehrere Fettbestandteile aus der Gruppe der $C_{12}$-$C_{30}$-Fettalkohole, der $C_{12}$-$C_{30}$-Fettsäuretriglyceride, der $C_{12}$-$C_{30}$-Fettsäuremonoglyceride, der $C_{12}$-$C_{30}$-Fettsäurediglyceride und/oder der Kohlenwasserstoffe enthält.

Als ganz besonders bevorzugte Fettbestandteile werden in diesem Zusammenhang die Bestandteile aus der Gruppe der $C_{12}$-$C_{30}$-Fettalkohole, der $C_{12}$-$C_{30}$-Fettsäuretriglyceride, der $C_{12}$-$C_{30}$-Fettsäuremonoglyceride, der $C_{12}$-$C_{30}$-Fettsäurediglyceride und/oder der Kohlenwasserstoffe verstanden. Im Sinne der vorliegenden Erfindung werden explizit nur nichtionische Substanzen als Fettbestandteile betrachtet. Geladene Verbindungen wie beispielsweise Fettsäuren und ihre Salze werden nicht als Fettbestandteil verstanden.

Bei den $C_{12}$-$C_{30}$-Fettalkoholen kann es sich um gesättigte, ein- oder mehrfach ungesättigte, lineare oder verzweigte Fettalkohole mit 12 bis 30 C-Atomen handeln.

Beispiele für bevorzugte lineare, gesättigte $C_{12}$-$C_{30}$-Fettalkohole sind Dodecan-1-ol (Dodecylalkohol, Laurylalkohol), Tetradecan-1-ol (Tetradecylalkohol, Myristylalkohol), Hexadecan-1-ol (Hexadecylalkohol, Cetylalkohol, Palmitylalkohol), Octadecan-1-ol (Octadecylalkohol, Stearylalkohol), Arachylalkohol (Eicosan-1-ol), Heneicosylalkohol (Heneicosan-1-ol) und/oder Behenylalkohol (Docosan-1-ol).

Bevorzugte lineare, ungesättigte Fettalkohole sind (9*Z*)-Octadec-9-en-1-ol (Oleylalkohol), (9*E*)-Octadec-9-en-1-ol (Elaidylalkohol), (9*Z*,12*Z*)-Octadeca-9,12-dien-1-ol (Linoleylalkohol), (9*Z*,12*Z*,15*Z*)-Octadeca-9,12,15-trien-1-ol (Linolenoylalkohol), Gadoleylalkohol ((9*Z*)-Eicos-9-en-1-ol), Arachidonalkohol ((5*Z*,8*Z*,11*Z*,14*Z*)-Eicosa-5,8,11,14-tetraen-1-ol), Erucylalkohol ((13*Z*)-Docos-13-en-1-ol) und/oder Brassidylalkohol ((13*E*)-Docosen-1-ol).

Die bevorzugten Vertreter für verzweigte Fettalkohole sind 2-Octyl-dodecanol, 2-Hexyl-Dodecanol und/oder 2-Butyl-dodecanol.

Im Rahmen einer weiteren bevorzugten Ausführungsform ist ein erfindungsgemäßes Mittel dadurch gekennzeichnet, dass es einen oder mehrere $C_{12}$-$C_{30}$-Fettalkohole aus der Gruppe aus Dodecan-1-ol (Dodecylalkohol, Laurylalkohol),

Tetradecan-1-ol (Tetradecylalkohol, Myristylalkohol),
Hexadecan-1-ol (Hexadecylalkohol, Cetylalkohol, Palmitylalkohol),
Octadecan-1-ol (Octadecylalkohol, Stearylalkohol),
Arachylalkohol (Eicosan-1-ol),
Heneicosylalkohol (Heneicosan-1-ol),
Behenylalkohol (Docosan-1-ol),
(9Z)-Octadec-9-en-1-ol (Oleylalkohol),

(9*E*)-Octadec-9-en-1-ol (Elaidylalkohol),
(9*Z*,12*Z*)-Octadeca-9,12-dien-1-ol (Linoleylalkohol),
(9*Z*,12*Z*,15*Z*)-Octadeca-9,12,15-trien-1-ol (Linolenoylalkohol),

Gadoleylalkohol ((9*Z*)-Eicos-9-en-1-ol),
Arachidonalkohol ((5*Z*,8*Z*,11*Z*,14*Z*)-Eicosa-5,8,11,14-tetraen-1-ol),
Erucylalkohol ((13*Z*)-Docos-13-en-1-ol),
Brassidylalkohol ((13*E*)-Docosen-1-ol),
2-Octyl-dodecanol,
2-Hexyl-Dodecanol und/oder

2-Butyl-dodecanol enthält.

Es hat sich als ganz besonders bevorzugt erwiesen, ein oder mehrere $C_{12}$-$C_{30}$-Fettalkohole in ganz bestimmten Mengenbereichen einzusetzen.

Es ist ganz besonders bevorzugt, wenn die das Mittel - bezogen auf das Gesamtgewicht des Mittelseinen oder mehrere $C_{12}$-$C_{30}$-Fettalkohole in einer Gesamtmenge von 2,0 bis 50,0 Gew.-%, bevorzugt von 3,0 bis 30,0 Gew.-%, weiter bevorzugt von 4,0 bis 20,0 Gew.-%, noch weiter bevorzugt von 5,0 bis 15,0 Gew.-% und ganz besonders bevorzugt von 5,0 bis 10,0 Gew.-% enthält.

Weiterhin als ganz geeigneten Fettbestandteil kann das Mittel auch mindestens ein $C_{12}$-$C_{30}$-Fettsäuretriglycerid, der $C_{12}$-$C_{30}$-Fettsäuremonoglycerid und/oder $C_{12}$-$C_{30}$-Fettsäurediglycerid enthalten. Unter einem $C_{12}$-$C_{30}$-Fettsäuretriglycerid wird im Sinne der vorliegenden Erfindung der Triester des dreiwertigen Alkohols Glycerin mit drei Äquivalenten Fettsäure verstanden. Dabei können sowohl strukturgleiche als auch unterschiedliche Fettsäuren innerhalb eines Triglyceridmoleküls an den Esterbildungen beteiligt sein.

Unter Fettsäuren sind erfindungsgemäß gesättigte oder ungesättigte, unverzweigte oder verzweigte, unsubstituierte oder substituierte $C_{12}$-$C_{30}$-Carbonsäuren zu verstehen. Ungesättigte Fettsäuren können einfach oder mehrfach ungesättigt sein. Bei einer ungesättigten Fettsäure kann bzw. können deren C-C-Doppelbindung(en) die Cis- oder Trans-Konfiguration aufweisen.

Es zeichnen sich die Fettsäuretriglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)- Tetracos-15-ensäure].

Die Fettsäuretriglyceride können auch natürlichen Ursprungs sein. Die in Sojaöl, Erdnußöl, Olivenöl, Sonnenblumenöl, Macadamianussöl, Moringaöl, Aprikosenkernöl, Marulaöl und/oder gegebenenfalls gehärtetem Rizinusöl vorkommenden Fettsäure-Triglyceride bzw. deren Gemische sind zum Einsatz im erfindungsgemäßen Produkt besonders geeignet.

Unter einem $C_{12}$-$C_{30}$-Fettsäuremonoglycerid wird der Monoester des dreiwertigen Alkohols Glycerin mit einem Äquivalent Fettsäure verstanden. Hierbei kann entweder die mittlere Hydroxygruppe des Glycerins oder die endständige Hydroxygruppe des Glycerins mit der Fettsäure verestert sein.

Es zeichnen sich die $C_{12}$-$C_{30}$-Fettsäuremonoglycerid durch besondere Eignung aus, bei welchen eine Hydroxygruppe des Glycerins mit einer Fettsäure verestert wird, wobei die Fettsäuren ausgewählt sind aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] oder Nervonsäure [(15Z)- Tetracos-15-ensäure].

Unter einem $C_{12}$-$C_{30}$-Fettsäurediglycerid wird der Diester des dreiwertigen Alkohols Glycerin mit zwei Äquivalenten Fettsäure verstanden. Hierbei können entweder die mittlere und eine endständige Hydroxygruppe des Glycerins mit zwei Äquivalenten Fettsäure verestert sein, oder aber beide endständigen Hydroxygruppen des Glycerins sind mit jeweils einer Fettsäure verestert. Das Glycerin kann hierbei sowohl mit zwei strukturgleichen als auch mit zwei unterschiedlichen Fettsäuren verestert sein.

Es zeichnen sich die Fettsäurediglyceride durch besondere Eignung aus, bei welchen mindestens eine der Estergruppen ausgehend von Glycerin mit einer Fettsäure ausgebildet wird, die ausgewählt wird aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure [(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)- Tetracos-15-ensäure].

Ganz besonders gute Ergebnisse wurden erhalten, wenn die Zusammensetzung (B) mindestens ein $C_{12}$-$C_{30}$-Fettsäuremonoglycerid enthielt, welches ausgewählt ist aus den Monoestern von Glycerin mit einem Äquivalent Fettsäure aus der Gruppe aus Dodecansäure (Laurinsäure), Tetradecansäure (Myristinsäure), Hexadecansäure (Palmitinsäure), Tetracosansäure (Lignocerinsäure), Octadecansäure (Stearinsäure), Eicosansäure (Arachinsäure), Docosansäure (Behensäure), Petroselinsäure [(Z)-6-Octadecensäure], Palmitoleinsäure [(9Z)-Hexadec-9-ensäure], Ölsäure

[(9Z)-Octadec-9-ensäure], Elaidinäsure [(9E)-Octadec-9-ensäure], Erucasäure [(13Z)-Docos-13-ensäure], Linolsäure [(9Z, 12Z)-Octadeca-9,12-diensäure, Linolensäure [(9Z,12Z,15Z)-Octadeca-9,12,15-triensäure, Elaeostearinäure [(9Z,11E,13E)-Octadeca-9,11,3-triensäure], Arachidonsäure [(5Z,8Z,11Z,14Z)-Icosa-5,8,11,14-tetraensäure] und/oder Nervonsäure [(15Z)- Tetracos-15-ensäure].

Im Rahmen einer weiteren Ausführungsform ist ein erfindungsgemäßes Mittel dadurch gekennzeichnet, dass es mindestens ein $C_{12}$-$C_{30}$-Fettsäuremonoglycerid enthält, welches ausgewählt ist aus den Monoestern von Glycerin mit einem Äquivalent Fettsäure aus der Gruppe aus Dodecansäure, Tetradecansäure, Hexadecansäure, Tetracosansäure, Octadecansäure, Eicosansäure und/oder Docosansäure.

Es hat sich als bevorzugt erwiesen, ein oder mehrere $C_{12}$-$C_{30}$-Fettsäuremono-, $C_{12}$-$C_{30}$-Fettsäuredi- und/oder $C_{12}$-$C_{30}$-Fettsäuretriglyceride in ganz bestimmten Mengenbereichen im Mittel einzusetzen.

Im Hinblick auf die Lösung der erfindungsgemäßen Aufgabenstellung hat es sich als vorteilhaft erwiesen, wenn das Mittel - bezogen auf das Gesamtgewicht des Mittels - ein oder mehrere $C_{12}$-$C_{30}$-Fettsäuremono-, $C_{12}$-$C_{30}$-Fettsäuredi- und/oder $C_{12}$-$C_{30}$-Fettsäuretriglyceride in einer Gesamtmenge von 0,1 bis 20,0 Gew.-%, bevorzugt 0,3 bis 15,0 Gew.-%, weiter bevorzugt 0,5 bis 10,0 Gew.-% und ganz besonders bevorzugt von 0,8 bis 5,0 Gew.-% enthielt.

Im Rahmen einer ganz besonders bevorzugten Ausführungsform ist ein erfindungsgemäßes Verfahren dadurch gekennzeichnet, dass das Mittel- bezogen auf das Gesamtgewicht des Mittelsein oder mehrere $C_{12}$-$C_{30}$-Fettsäuremono-, $C_{12}$-$C_{30}$-Fettsäuredi- und/oder $C_{12}$-$C_{30}$-Fettsäuretriglyceride (b2) in einer Gesamtmenge von 0,1 bis 20,0 Gew.-%, bevorzugt 0,3 bis 15,0 Gew.-%, weiter bevorzugt 0,5 bis 10,0 Gew.-% und ganz besonders bevorzugt von 0,8 bis 5,0 Gew.-% enthält.

Die $C_{12}$-$C_{30}$-Fettsäuremono-, $C_{12}$-$C_{30}$-Fettsäuredi- und/oder $C_{12}$-$C_{30}$-Fettsäuretriglyceride können als alleinige Fettbestandteile in den Mitteln eingesetzt werden. Es kann sich jedoch auch erfindungsgemäß, mindestens ein $C_{12}$-$C_{30}$-Fettsäuremono-, $C_{12}$-$C_{30}$-Fettsäuredi- und/oder $C_{12}$-$C_{30}$-Fettsäuretriglycerid in Kombination mit mindestens einem $C_{12}$-$C_{30}$-Fettalkohol in das Mittel einzuarbeiten.

Weiterhin als ganz besonders bevorzugten Fettbestandteil kann das Mittel auch mindestens einen Kohlenwasserstoff enthalten.

Kohlenwasserstoffe sind ausschließlich aus den Atomen Kohlenstoff und Wasserstoff bestehende Verbindungen mit 8 bis 80 C-Atomen. Bevorzugt sind in diesem Zusammenhang insbesondere aliphatische Kohlenwasserstoffe wie beispielsweise Mineralöle, flüssige Paraffinöle (z.B. Paraffinium Liquidum oder Paraffinum Perliquidum), Isoparaffinöle, halbfeste Paraffinöle, Paraffinwachse, Hartparaffin (Paraffinum Solidum), Vaseline und Polydecene.

Als besonders geeignet haben sich in diesem Zusammenhang flüssige Paraffinöle (Paraffinum Liquidum und Paraffinium Perliquidum) erwiesen. Ganz besonders bevorzugt handelt es sich bei dem Kohlenwasserstoff um Paraffinum Liquidum, auch Weißöl genannt. Bei Paraffinum Liquidum handelt es sich um ein Gemisch gereinigter, gesättigter, aliphatischer Kohlenwasserstoffe, das größtenteils aus Kohlenwasserstoffketten mit einer C-Kettenverteilung von 25 bis 35 C-Atomen besteht.

Ganz besonders gute Ergebnisse wurden erhalten, wenn das Mittel mindestens einen Kohlenwasserstoff enthielt, der ausgewählt ist aus der Gruppe der Mineralöle, der flüssige Paraffinöle, Isoparaffinöle, halbfeste Paraffinöle, Paraffinwachse, Hartparaffin (Paraffinum Solidum), Vaseline und Polydecene.

Im Rahmen einer ganz besonders bevorzugten Ausführungsform ist ein erfindungsgemäßes Mittel dadurch gekennzeichnet, dass es mindestens einen Fettbestandteil aus der Gruppe der Kohlenwasserstoffe enthält.

Im Hinblick auf die Lösung der erfindungsgemäßen Aufgabenstellung hat es sich als ganz besonders bevozugt erwiesen, wenn das Mittel - bezogen auf das Gesamtgewicht des Mittels - einen oder mehrere Kohlenwasserstoffe in einer Gesamtmenge von 0,5 bis 20,0 Gew.-%, bevorzugt 0,7 bis 10,0 Gew.-%, weiter bevorzugt von 0,9 bis 5,0 Gew.-% und ganz besonders bevorzugt von 1,0 bis 4,0 Gew.-% enthielt.

Im Rahmen einer ganz besonders bevorzugten Ausführungsform ist ein erfindungsgemäßes Mittel dadurch gekennzeichnet, dass es - bezogen auf das Gesamtgewicht des Mittels - einen oder mehrere Kohlenwasserstoffe in einer Gesamtmenge von in einer Gesamtmenge von 0,5 bis 20,0 Gew.-%, bevorzugt 0,7 bis 10,0 Gew.-%, weiter bevorzugt von 0,9 bis 5,0 Gew.-% und ganz besonders bevorzugt von 1,0 bis 4,0 Gew.-% enthält.

Der oder die Kohlenwasserstoffe können als alleinige Fettbestandteile (a4) in den Mitteln eingesetzt werden. Es ist jedoch ebenfalls erfindungsgemäß, mindestens einen Kohlenwasserstoff in Kombination mit mindestens einem weiteren Bestandteil in die Mittel einzuarbeiten.

Ganz besonders bevorzugt enthält das Mittel mindestens einen Fettbestandteil (a4) aus der Gruppe der $C_{12}$-$C_{30}$-Fettalkohole und mindestens einen weiteren Fettbestandteil aus der Gruppe der Kohlenwasserstoffe.

<u>Wassergehalt im Mittel</u>

Bei dem zuvor beschriebenen Mittel handelt es sich um ein anwendungsbereites Mittel, welches auf das keratinische Material appliziert werden kann. Dieses anwendungsbereite Mittel besitzt bevorzugt einen hohen Wasseranteil. Es hat sich herausgestellt, dass besonders die Mittel besonders gut geeignet sind, die - bezogen auf das

Gesamtgewicht des Mittels - 50,0 bis 98,0 Gew.-%, bevorzugt 60,0 bis 90,0 Gew.-%, weiter bevorzugt 70,0 bis 90,0 Gew.-% und ganz besonders bevorzugt 75,0 bis 90,0 Gew.-% Wasser enthalten.

In einer weiteren explizit ganz besonders bevorzugten Ausführungsform ist ein erfindungsgemäßes Mittel dadurch gekennzeichnet, dass es - bezogen auf das Gesamtgewicht des Mittels - 50,0 bis 98,0 Gew.-%, bevorzugt 60,0 bis 90,0 Gew.-%, weiter bevorzugt 70,0 bis 90,0 Gew.-% und ganz besonders bevorzugt 75,0 bis 90,0 Gew.-% Wasser enthält.

<u>weitere optionale Inhalttstoffe im Mittel</u>

Zusätzlich zu den bereits beschriebenen erfindungswesentlichen Bestandteilen (a1) bis (a4) kann das Mittel zusätzlich auch noch weitere optionale Inhaltsstoffe enthalten.

So kann das Mittel beispielsweise ein filmbildendes Polymer enthalten. Das filmbildende Polymer kann zum Beispiel ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/EthylenCopolymeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren, explizit ganz besonders bevorzugt Polyvinylpyrrolidon (PVP).

Weitere geeignete filmbildende Polymere können augewählt sein aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Es haben sich insbesondere die filmbildenden Polymere als gut geeignet erwiesen, die aus der Gruppe der synthetischen Polymere, der durch radikalische Polymerisation erhältlichen Polymere oder der natürlichen Polymere ausgewählt werden.

Weitere besonders gut geeignete filmbildende Polymere können ausgewählt werden aus den Homopolymere oder Copolymeren von Olefinen, wie beispielsweise Cycloolefinen, Butadien, Isopren oder Styren, Vinylethern, Vinylamiden, den Estern oder Amiden von (Meth)Acrylsäure mit mindestens einer $C_1$-$C_{20}$-Alkylgruppe, einer Arylgruppe oder einer C2-C10-Hydroxyalkylgruppe.

Weitere filmbildende Polymere können ausgewählt sein aus den Homo- oder Copolymeren von Isooctyl-(meth) acrylat; Isononyl-(meth)acrylat; 2-Ethylhexyl-(meth)acrylat; Lauryl-(meth)acrylat); isopentyl-(meth)acrylat; n-Butyl-(meth)acrylat); Isobutyl-(meth)acrylat; Ethyl-(meth)acrylat; Methyl-(meth)acrylat; tert-Butyl-(meth)acrylat; Stearyl-(meth)acrylat; Hydroxyethyl-(meth)acrylat; 2-Hydroxypropyl-(methacrylat; 3-Hydroxypropyl-(meth)acrylat und/oder Gemischen hiervon.

Weitere filmbildende Polymere können ausgewählt sein aus den Homo- oder Copolymeren von (Meth)acrylamid; N-Alkyl-(meth)acrylamiden, insbesondere solchen mit C2-C18 Alkylgruppen, wie beispielsweise N-Ethyl-acrylamid, N-tert-butyl-acrylamid, le N-Octyl-crylamid; N-Di(C1-C4)alkyl-(meth)acrylamid.

Weitere geeignete anionische Copolymere sind beispielsweise Copolymere aus Acrylsäure, Methacrylsäure oder deren $C_1$-$C_6$-Alkylestern, wie sie unter der INCI-Deklaration Acrylates Copolymere vertrieben werden. Ein geeignetes Handelsprodukt ist beispielsweise Aculyn® 33 der Firma Rohm & Haas. Weiterhin bevorzugt sind aber auch Copolymere aus Acrylsäure, Methacrylsäure oder deren $C_1$-$C_6$-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20.

Auf dem Markt befindliche Polymere sind beispielsweise Aculyne 22 (Acrylates/Steareth-20 Methacrylate Copolymer), Aculyne 28 (Acrylates/Beheneth-25 Methacrylate Copolymer), Structure 2001®(Acryla-tes/Steareth-20 Itaconate Copolymer), Structure 3001®(Acrylates/Ceteth-20 Itaconate Copolymer), Structure Plus®(Acrylates/Aminoacrylates C10-30 Alkyl PEG-20 Itaconate Copolymer), Carbopol® 1342, 1382, Ultrez 20, Ultrez 21 (Acrylates/C10-30 Alkyl Acrylate Crosspolymer), Synthalen W 2000® (Acrylates/Palmeth-25 Acrylate Copolymer) oder das Rohme und Haas vertriebene Soltex OPT (Acrylates/C12-22 Alkyl methacrylate Copolymer).

Als geeignete Polymere auf der Basis von Vinyl-Monomeren können beispielsweise genannt werden die Homo- und Copolymere des N-Vinylpyrrolidons, des Vinylcaprolactams, des Vinyl-(C1-C6-)alkyl-Pyrrols, des Vinyl-oxazols, des Vinyl-thiazols, des Vinylpyrimidins, des Vinylimidazols.

Weiterhin geeignet sind die Copolymere Octylacrylamid/acrylates/ butylaminoethyl-methacrylate copolymer, wie es beispielsweise unter den Handelsnamen AMPHOMER® ou LOVOCRYL® 47 von NATIONAL STARCH kommerziell vertrieben wird, oder auch die Copolymere von Acrylates/Octylacrylamide die unter den Handelsnamen DERMACRYL® LT und DERMACRYL® 79 von NATIONAL STARCH vertrieben werden.

Als geeignete Polymere auf der Basis von Olefinen können beispielsweise genannt werden die Homo- und Copolymere des Ethylens, des Propylens, des Butens, des Isoprens und des Butadiens.

Im Rahmen einer weiteren Ausführungsform können als filmbildenden hydrophoben Polymere die Block-Copoylmere eingesetzt werden, die mindestens einen Block aus Styren oder den Derivaten des Styrens umfassen. Bei diesen Block-Copolymeren kann es sich um Copolymere handeln, die neben einem Styren-Block einen oder mehrere weitere Blöcke enthalten, wie beispielsweise Styren/Ethylen, Styren/Ethylen/Butylen, Styen/Butylen, Styren/Isopren, Styren/Butadien. Entsprechende Polymere werden von der BASF unter dem Handelsnamen "Luvitol HSB" kommerziell vertrieben.

Wenn im erfindungsgemäßen Mittel prinzipiell auch sowohl anionische als auch kationische und/oder nichtionische Polymere eingesetzt werden können, so hat es sich als ganz besonders bevorzugt erwiesen, weitere ionische Verbindungen nicht oder nur in geringen Mengen einzusetzen. Mit anderen Worten konnte insbesondere dann eine besonders starke Verbesserung der Farbintensität erzielt werden, wenn das Mittel eine vorwiegend nichtionische Basis darstellte und daher kationische und anionische Polymere entweder gar nicht oder nur in sehr geringen Mengen enthielt. Aus diesem Grund ist hat es sich als ganz besonders bevorzugt herausgestellt, wenn der Gesamtgehalt aller im Mittel enthaltenen anionischen Polymer unterhalb von 0,1 Gew.-% liegt. Weiterhin hat es als ganz besonders bevorzugt herausgestellt, wenn der Gesamtgehalt aller im Mittel enthaltenen kationischen Polymeren unterhalb von 0,1 Gew.-% liegt. Der Mengenanteil an katonischen bzw. anionischem Polymer ist hierbei jeweils auf das Gesamtgewicht des Mittels bezogen.

In einer weiteren ganz besonders bevorzugten Ausführungsform ist ein erfindungsgemäßes Mittel dadurch gekennzeichnet, dass - bezogen auf das Gesamgtewicht des Mittels -

- der Gesamtgehalt aller im Mittel enthaltenen anionischen Polymere unterhalb von 0,1 Gew.-% liegt, und
- der Gesamtgehalt aller im Mittel enthaltenen kationischen Polymere unterhalb von 0,1 Gew.-% liegt.

Neben den zuvor beschriebenen nichtionischen Tensiden können die Mittel prinzipiell zusätzlich auch noch ein oder mehrere geladene Tenside enthalten. Unter dem Begriff Tenside werden grenzflächenaktive Substanzen verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Als zwitterionische Tenside werden solche oberflächenaktiven Verbindungen bezeichnet, die im Molekül mindestens eine quartäre Ammoniumgruppe und mindestens eine $-COO^{(-)}$ - oder $-SO_3^{(-)}$ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethylimidazoline mit jeweils 8 bis 18 C-Atomen in der Alkyl- oder Acylgruppe sowie das Kokosacylaminoethylhydroxyethylcarbo xymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer $C_8$ - $C_{24}$ - Alkyl- oder -Acylgruppe im Molekül mindestens eine freie Aminogruppe und mindestens eine -COOH- oder $-SO_3H$-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine.

Beispiele für ampholytische Tenside sind das N-Kokosalkylaminopropionat, das Kokosacylaminoethylaminopropionat und das $C_{12}$ - $C_{18}$ - Acylsarcosin.

Weiterhin können die Mittel zusätzlich auch mindestens ein kationisches Tensid enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Beispiele für kationische Tenside sind

- quartäre Ammoniumverbindungen, die als hydrophobe Reste ein oder zwei Alkylketten mit einer Kettenlänge von 8 bis 28 C-Atomen tragen können,
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein. Neben der funktionellen Einheit, welche die kationische Ladung trägt, kann das kationische Tensid auch weitere ungeladene funktionelle Gruppen beinhalten, wie dies beispielsweise bei Esterquats der Fall ist. Die kationischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Weiterhin können die erfindungsgemäßen Mittel auch mindestens ein anionisches Tensid enthalten. Als anionische Tenside werden oberflächenaktive Mittel mit ausschließlich anionischen Ladungen (neutralisiert durch ein entsprechendes Gegenkation) bezeichnet. Beispiele für anionische Tenside sind Fettsäuren, Alkylsulfate, Alkylethersulfate und Ethercarbonsäuren mit 12 bis 20 C-Atomen in der Alkylgruppe und bis zu 16 Glykolethergruppen im Molekül.

Wenn im erfindungsgemäßen Mittel prinzipiell auch sowohl anionische als auch kationische und/oder nichtionische Tenside eingesetzt werden können, so hat es sich als ganz besonders bevorzugt erwiesen, weitere ionische Verbindungen nicht oder nur in geringen Mengen einzusetzen. Mit anderen Worten konnte insbesondere dann eine besonders starke Verbesserung der Farbintensität erzielt werden, wenn das Mittel eine vorwiegend nichtionische Basis darstellte und daher kationische und anionische Tenside entweder gar nicht oder nur in sehr geringen Mengen enthielt. Aus diesem Grund ist hat es sich als ganz besonders bevorzugt herausgestellt, wenn der Gesamtgehalt aller im Mittel enthaltenen anionischen Tenside unterhalb von 0,1 Gew.-% liegt. Weiterhin hat es als ganz besonders bevorzugt herausgestellt, wenn der Gesamtgehalt aller im Mittel enthaltenen kationischen Tenside unterhalb von 0,1 Gew.-% liegt. Der Mengenanteil an katonischen bzw. anionischem Tensid ist hierbei jeweils auf das Gesamtgewicht des Mittels bezogen.

In einer weiteren ganz besonders bevorzugten Ausführungsform ist ein erfindungsgemäßes Mittel dadurch gekennzeichnet, dass - bezogen auf das Gesamgtewicht des Mittels -

- der Gesamtgehalt aller im Mittel enthaltenen anionischen Tenside unterhalb von 0,1 Gew.-% liegt, und
- der Gesamtgehalt aller im Mittel enthaltenen kationischen Tenside unterhalb von 0,1 Gew.-% liegt.

Die Mittel können ferner auch noch weitere Wirk-, Hilfs- und Zusatzstoffe enthalten, wie beispielsweise Lösungsmittel, Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, $N_2O$, Dimethylether, $CO_2$ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

<u>pH-Wert der Mittel</u>

Die pH-Werte des erfindungsgemäßen Mittels wird bevorzugt auf einen neutral bis alkalischen pH-Wert eingestellt. Ganz besonders bevorzugt besitzt das Mittel einen alkalischen pH-Wert im Bereich von 7,0 bis 11,5 bevorzugt von 8,0 bis 11,0, und besonders bevorzugt von 8,5 bis 10,5. Unter basischen Bedingungen kann das aminofunktionalisierte Silikonpolymer (a1) besonders gut und ohne Protonierung gelöst bzw. dispergiert werden.

Im Rahmen einer weiteren bevorzugten Ausführungsform ist ein erfindungsgemäßes Mittel dadurch gekennzeichnet, dass es einen pH-Wert von 7,0 bis 11,5 bevorzugt von 8,0 bis 11,0, und besonders bevorzugt von 8,5 bis 10,5 besitzt.

Zur Einstellung des gewünschten pH-Wertes kann das Mittel (a) und/oder (b) mindestens ein Alkalisierungsmittel enthalten. Bei den pH-Werten im Sinne der vorliegenden Erfindung handelt es sich um pH-Werte, die bei einer Temperatur von 22°C gemessen wurden.

Als Alkalisierungsmittel können die Mittel beispielsweise Ammoniak, Alkanolamine und/oder basische Aminosäuren enthalten.

Die in dem erfindungsgemäßen Mittel einsetzbaren Alkanolamine werden bevorzugt ausgewählt aus primären Aminen mit einem $C_2$-$C_6$-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Erfindungsgemäß besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das erfindungsgemäße Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -$SO_3$H-Gruppe enthält. Bevorzugte Aminosäuren sind Aminocarbonsäuren, insbesondere $\alpha$-(alpha)-Aminocarbonsäuren und $\omega$-Aminocarbonsäuren, wobei $\alpha$-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische $\alpha$-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein erfindungsgemäßes Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus kann das Mittel weitere Alkalisierungsmittel, insbesondere anorganische Alkalisierungsmittel enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

In einer weiteren ganz besonders bevorzugten Ausführungsform ist ein erfindungsgemäßes Verfahren dadurch gekennzeichnet, dass das Färbemittel (a) mindestens ein Alkalisierungsmittel aus der Gruppe aus Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methyl-propan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat enthält.

Verfahren zum Färben von Keratinmaterial

Die zuvor beschriebenen Mittel lassen sich hervorragend in Verfahren zum Färben von keratinischem Material, insbesondere von menschlichen Haaren einsetzen.

Ein zweiter Gegenstand der vorliegenden Erfindung ist daher ein Verfahren zum Färben von keratinischem Material, insbesondere menschlichen Haaren, umfassend die folgenden Schritte:

(1) Anwendung eines Färbemittels auf dem keratinischem Material, wobei das Färbemittel ein Mittel ist, wie es bei der Beschreibung der ersten Erfindungsgegenstand im Detail offenbart wurde,

(2) Einwirken des Färbemittels auf dem keratinischen Material und
(3) Ausspülen des Färbemittels mit Wasser.

In Schritt (1) des erfindungsgemäßen Verfahrens wird das Mittel des ersten Erfindungsgegenstand auf dem keratinische Material, bei dem es sich ganz besonders bevorzugt um menschliche Haare handelt, angewendet.

In Schritt (2) des erfindungsgemäßen Verfahrens wird das Mittel dann nach seiner Applikation auf das keratinische Material einwirken gelassen. In diesem Zusammenhang sind verschiedene Einwirkzeiten von beispielsweise 30 Sekunden bis 60 Minuten denkbar.

Ein großer Vorteil des erfindungsgemäßen Färbesystems liegt jedoch darin, dass auch in sehr kurzen Zeiträumen nach kurzen Einwirkzeiten ein intensive Farbergebnis erzielt werden kann. Aus diesem Grund ist es von Vorteil, wenn die Anwendungsmischung nach ihrer Applikation nur für vergleichsweise kurze Zeiträume von 30 Sekunden bis 15 Minuten, bevorzugt von 30 Sekunden bis 10 Minuten, und besonders bevorzugt von 1 bis 5 Minuten auf dem Keratinmaterial verbleibt.

In einer weiteren bevorzugten Ausführungsform ist ein erfindungsgemäßes Verfahren gekennzeichnet durch das

(2) Einwirken des Färbemittels auf dem keratinischen Material für einen Zeitraum von 30 Sekunden bis 15 Minuten, bevorzugt von 30 Sekunden bis 10 Minuten, und besonders bevorzugt von 1 bis 5 Minuten.

Im Anschluss an das Einwirken der Anwendungsmischung auf das Keratinmaterial wird diese schließlich in Schritt (3) des Verfahrens mit Wasser ausgespült.

Hierbei kann die Awendungsmischung in einer Ausführungsform nur mit Wasser, d.h. ohne Zuhilfenahme eines Nachbehandlungsmittels oder eines Shampoos, ausgewaschen werden. Auch die Anwendung eines Nachbehandlungsmittels oder Conditioners in Schritt (6) ist prinzipiell denkbar.

Zur Lösung der erfindungsgemäßen Aufgabenstellung und zur Erhöhung des Anwendungskomforts hat es sich jedoch als ganz besonders bevorzugt herausgestellt, das Ausspülen des Mittels in Schritt (3) ausschließlich mit Wasser ohne Zuhilfenahme eines weiteren Nachbehandlungsmittels, Shampoos oder Conditioners vorzunehmen.

In einer weiteren bevorzugten Ausführungsform ist ein erfindungsgemäßes Verfahren gekennzeichnet durch das

(3) Ausspülen des Färbemittels ausschließlich mit Wasser.

Betreffend die weiteren bevorzugten Ausführungsformen der erfindungsgemäßen Verfahrens gilt *mutatis mutantis* das zum erfindungsgemäßen Mittel gesagte.

Beispiele

1. Formulierungen

Es wurden die folgenden Formulierungen hergestellt (alle Angaben, sofern nichts anderes angegeben ist, in Gew.-%):

| Färbemittel | (V1) | (E1) |
|---|---|---|
| Cetylalkohol | 3,6 | 3,6 |
| Stearylalkohol | 2,1 | 2,1 |
| Paraffinum Liquidum | 2,1 | 2,1 |
| Ceteareth-30 (Cetearylalkohol, ethoxylated 30 EO) | 1,2 | 1,2 |
| Brij S 100 PA SG (Stearylalkohol, ethoyliert 100 EO, Croda) | 0,6 | 0,6 |
| Cutina GMS V (INCI: Glyceryl stearate, Glyceol Mono/dipalmitate/stearate) CAS-Nr. 85251-77-0 | 0,6 | 0,6 |
| 1,2-Propandiol | 6,3 | 6,3 |
| Lavanya Zuni (organisches Pigment,Neelikon Red, 111P0200, CI 12490) | 1,0 | 1,0 |
| Dow Corning 2-8566 (Siloxanes and Silicones, 3-[(2-Aminoethyl)amino]-2-methylpropyl Me, Di-Me-Siloxane" | 2,5 | 2,5 |
| Ammoniak (25%ige wässrige Lösung) | 0,20 | 0,20 |
| 2-Phenoxyethanol | --- | 0,30 |
| 4-Hydroxybenzoesäuremethylester | --- | 0,40 |

(fortgesetzt)

| Färbemittel | (V1) | (E1) |
|---|---|---|
| Wasser | ad 100 | ad 100 |

2. Anwendung

Das Mittel (E1) wurde auf Haarsträhnen (Kerling, Euronaturhaar weiß, Flottenverhältnis: 1 g Mittel (E1) pro g Haarsträhne) appliziert. Das Mittel wurde für drei Minuten einwirken gelassen. Im Anschluss daran wurden die Haarsträhne gründlich (1 Minute) mit Wasser ausgewaschen, getrocknet und dann farbmetrisch mit einem Farbmessgerät der Firma Datacolor, Typ Spectraflash 450 vermessen.

Als Vergleich wurde die Vergleichsformulierung V1 auf Haarsträhnen (Kerling, Euronaturhaar weiß, Flottenverhältnis: 1 g Mittel (V1) pro g Haarsträhne) appliziert und drei Minuten einwirken gelassen.

Im Anschluss daran wurden auch diese Haarsträhnen gründlich (1 Minute) mit Wasser ausgewaschen, getrocknet und farbmetrisch vermessen.

Nach der ersten farmetrischen Vermessung wurden alle gefärbten Strähnen 5 mal von derselben Person mit der Hand gewaschen (pro Haarschwäsche: Auftrag von jeweils 0,25 g Shampoo (Schauma, 7-Kräuter) auf die angefeuchtete Strähne, 30 min shampoonieren, 1 min mit Wasser auswaschen, trocknen). Nach 5 Haarwäschen wurde jede Strähne erneut farbmetrisch vermessen.

Der für die Beurteilung der Waschechtheit herangezogene dE-Wert ergibt sich aus den am jeweiligen Strähnenteil gemessenen L*a*b*-Farbmesswerten wie folgt:

$$dE = [\ (L_i - L_0)^2 + (a_i - a_0)^2 + (b_i - b_0)]^{1/2}$$

$L_0$, $a_0$ und $b_0$ = Messwerte vor der Haarwäsche
$L_i$, $a_i$ und $b_i$ = Messwerte nach der Haarwäsche

Die Buntheit einer Färbung (Chroma) berechnet sich nach der Formel

$$C = \sqrt{a^2 + b^2}$$

Je größer der C-Werst ist, desto höher ist die Buntheit einer Färbung.

| Mittel | L | a | b | Chroma C | dE |
|---|---|---|---|---|---|
| Vergleich (V1) 0 Haarwäschen | 33,88 | 46,95 | 22,10 | 51,98 | 40,8 |
| Vergleich (V1) 5 Haarwäschen | 63,50 | 19,24 | 17,76 | 26,19 | |
| Erfindung (E1) 0 Haarwäschen | 35,76 | 47,93 | 21,39 | 52,49 | 2,2 |
| Erfindung (E1) 5 Haarwäschen | 36,60 | 46,45 | 19,95 | 50,55 | |

Wurden die vor und nach 5 Haarwäschen erhaltenen Farbmetrikwerte miteinander verglichen, so zeigten die mit der Vergleichsformulierung erhaltenen Färbungen einen größeren Farbabstand und damit eine schlechtere Waschechtheit. Die mit der erfindungsgemäßen Formulierung erhaltenen Färbungen besaßen eine bessere Waschechtheit.

Nach 0 Haarwäschen war der mit der erfindungsgemäßen Formulierung erhaltene Chroma-Wert im Vergleich zur Vergleichsformulierung leicht erhöht. Nach 5 Haarwäschen war der mit der erfindungsgemäßen Formulierung erhaltene Chroma-Wert sehr viel höher als der mit der Vergleichsformulierung erhaltene Chroma-Wert.

**Patentansprüche**

1. Mittel zum Färben von keratinischem Material, insbesondere menschlichen Haaren, enthaltend

(a1) mindestens ein aminofunktionalisiertes Silikonpolymer, das Struktureinheiten der Formel (Si-I) und der Formel (Si-II) umfasst

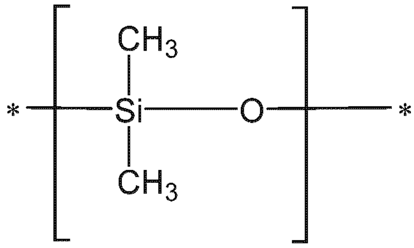 (Si-I) 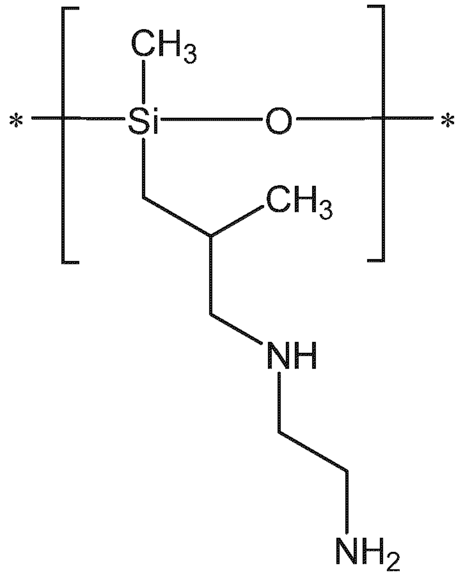 (Si-II),

und
(a2) mindestens eine farbgebende Verbindung, und
(a3) mindestens ein Konservierungsmittel aus der Gruppe aus 2-Phenoxyethanol, 4-Hydroxybenzoesäuremethylester, 4-Hydroxybenzoesäuerpropylester, Benzylalkohol, 1-Phenoxy-propan-2-ol, Isopropanol, Ethanol, Zinkpyrithion, Benzoesäure, Salicylsäure, Sorbinsäure, Ameisensäure, Propionsäure, 2-Hydroxydiphenyl, 4-Hydroxybenzoesäure, Dehydracetsäure, Dibromhexamidin, 10-Undecylensäure, Hexetidinum, Trichlorcarban, Triclosanum, 4-Chlor-3,4-dimethylphenol, Imidazolidinylharnstoff, Hexamethylentetramin, 1-(4-Chlorphenoxy)-1-(imidazol-1-yl)-3,3-dimethyl-2-butanon, 1,3-Bis-(hydroxymethyl)-5,5-dimethyl-2,4-imidazolidindion, 1-Hydroxy-4-methyl-6-(2,4,4-trimethylpentyl)-2-pyridon, Bromchlorophen, 3-Methyl-4-(1-methylethyl)phenol, 5-Chlor-2-methyl-3(2H)-isothiazolon, 2-Benzyl-4-chlorphenol, 2-Chloracetamid, Chlorhexidin, 4,4-Dimethyl-1,3-oxazolidin, Hexamidinum, 5-Ethyl-1-aza-3,7-dioxabicyclo-[3.3.0]-octan, Chlorphenesin, Natriumhydroxymethyl-aminoacetat, Benzylhemiformal, 3-Iod-2-propinyl-butylcarbamat, Methylisothiazolinon und Ethyl Lauroyl Arginat, und
(a4) mindestens ein nichtionisches Tensid aus der Gruppe der Anlagerungsprodukte von Ethylenoxid an $C_8$-$C_{24}$-Fettalkohole.

2. Mittel nach Anspruch 1, **dadurch gekennzeichnet, dass** es - bezogen auf das Gesamtgewicht des Mittels - ein oder mehrere aminofunktionalisierte Silikonpolymere (a1) in einer Gesamtmenge von 0,1 bis 8,0 Gew.-%, bevorzugt 0,2 bis 5,0 Gew.-%, weiter bevorzugt von 0,3 bis 3,0 Gew.-% und ganz besonders bevorzugt von 0,4 bis 2,5 Gew.-% enthält.

3. Mittel nach einem der Ansprüche 1 bis 2, **dadurch gekennzeichnet, dass** es mindestens eine farbgebende Verbindung (a2) aus der Gruppe der Pigmente, der direktziehenden Farbstoffe, der photochromen Farbstoffe und der thermochromen Farbstoffe enthält.

4. Mittel nach einem der Ansprüche 1 bis 3, **dadurch gekennzeichnet, dass** es mindestens eine farbgebende Verbindung (a2) aus der Gruppe der anorganischen Pigmente enthält, die bevorzugt ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

5. Mittel nach einem der Ansprüche 1 bis 4, **dadurch gekennzeichnet, dass** es mindestens eine farbgebende Verbindung (a2) aus der Gruppe der organischen Pigmente enthält, die bevorzugt ausgewählt ist aus der Gruppe aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470.

6. Mittel nach einem der Ansprüche 1 bis 5, **dadurch gekennzeichnet, dass** es - bezogen auf das Gesamtgewicht des Mittels (a) - ein oder mehrere Pigmente (a2) in einer Gesamtmenge von 0,01 bis 10,0 Gew.-%, bevorzugt 0,1 bis 5,0 Gew.-%, weiter bevorzugt von 0,2 bis 2,5 Gew.-% und ganz besonders bevorzugt von 0,25 bis 1,5 Gew.-% enthält.

7. Mittel nach einem der Ansprüche 1 bis 6, **dadurch gekennzeichnet, dass** es (a3) 2-Phenoxyethanol und/oder 4-Hydroxybenzoesäuremethylester enthält.

8. Mittel nach einem der Ansprüche 1 bis 7, **dadurch gekennzeichnet, dass** es - bezogen auf das Gesamtgewicht des Mittels - ein oder mehrere Konservierungsmittel (a3) in einer Gesamtmenge von 0,01 bis 5,0 Gew.-%, bevorzugt 0,1 bis 2,5 Gew.-%, weiter bevorzugt von 0,15 bis 1,0 Gew.-% und ganz besonders bevorzugt von 0,2 bis 0,8 Gew.-% enthält.

9. Mittel nach einem der Ansprüche 1 bis 8, **dadurch gekennzeichnet, dass** es mindestens ein nichtionisches Tensid (a4) der Formel (T-I) enthält,

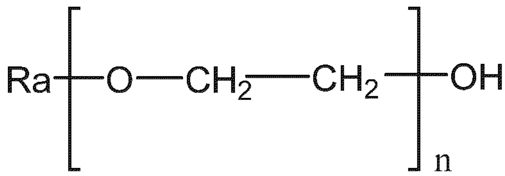

(T-I)

worin

Ra für eine gesättigte oder ungesättigte, unverzweigte oder verzweigte $C_8$-$C_{24}$-Alkylgruppe, bevorzugt für eine gesättigte, unverzweigte $C_{16}$- bis $C_{18}$- Alkylgruppe, steht und
n für eine ganze Zahl von 80 bis 120, bevorzugt für eine ganze Zahl von 90 bis 110 und besonders bevorzugt für die Zahl 100, steht.

10. Mittel nach einem der Ansprüche 1 bis 9, **dadurch gekennzeichnet, dass** es mindestens ein nichtionisches Tensid (a4) der Formel (T-II) enthält,

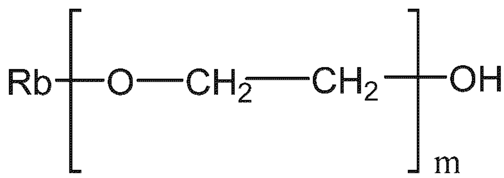

(T-II)

worin

Rb für eine gesättigte oder ungesättigte, unverzweigte oder verzweigte $C_8$-$C_{24}$-Alkylgruppe, bevorzugt für eine gesättigte, unverzweigte $C_{16}$- bis $C_{18}$- Alkylgruppe, steht und
m für eine ganze Zahl von 10 bis 40, bevorzugt für eine ganze Zahl von 20 bis 35 und besonders bevorzugt für die Zahl 30, steht.

11. Mittel nach einem der Ansprüche 1 bis 10, **dadurch gekennzeichnet, dass** es einen oder mehrere Fettbestandteile

aus der Gruppe der $C_{12}$-$C_{30}$-Fettalkohole, der $C_{12}$-$C_{30}$-Fettsäuretriglyceride, der $C_{12}$-$C_{30}$-Fettsäuremonoglyceride, der $C_{12}$-$C_{30}$-Fettsäurediglyceride und/oder der Kohlenwasserstoffe enthält.

**12.** Mittel nach einem der Ansprüche 1 bis 11, **dadurch gekennzeichnet, dass** - bezogen auf das Gesamtgewicht des Mittels -

- der Gesamtgehalt aller im Mittel enthaltenen anionischen Tenside unterhalb von 0,1 Gew.-% liegt, und
- der Gesamtgehalt aller im Mittel enthaltenen kationischen Tenside unterhalb von 0,1 Gew.-% liegt.

**13.** Mittel nach einem der Ansprüche 1 bis 12, **dadurch gekennzeichnet, dass** - bezogen auf das Gesamtgewicht des Mittels -

- der Gesamtgehalt aller im Mittel enthaltenen anionischen Polymere unterhalb von 0,1 Gew.-% liegt, und
- der Gesamtgehalt aller im Mittel enthaltenen kationischen Polymere unterhalb von 0,1 Gew.-% liegt.

**14.** Mittel nach einem der Ansprüche 1 bis 13, **dadurch gekennzeichnet, dass** es Wasser enthält und einen pH-Wert von 7,0 bis 11,5 bevorzugt von 8,0 bis 11,0, und besonders bevorzugt von 8,5 bis 10,5 besitzt.

**15.** Verfahren zum Färben von keratinischem Material, insbesondere menschlichen Haaren, umfassend die folgenden Schritte:

(1) Anwendung eines Färbemittels nach einem der Ansprüche 1 bis 14 auf dem keratinischem Material,
(2) Einwirken des Färbemittels auf dem keratinischen Material und
(3) Ausspülen des Färbemittels mit Wasser.

**16.** Verfahren nach Anspruch 15, **gekennzeichnet durch** das
(2) Einwirken des Färbemittels (a) auf dem keratinischen Material für einen Zeitraum von 30 Sekunden bis 15 Minuten, bevorzugt von 30 Sekunden bis 10 Minuten, und besonders bevorzugt von 1 bis 5 Minuten.

**Claims**

**1.** An agent for dyeing keratinous material, in particular human hair, containing

(a1) at least one amino-functionalized silicone polymer comprising structural units of formula (Si-I) and formula (Si-II)

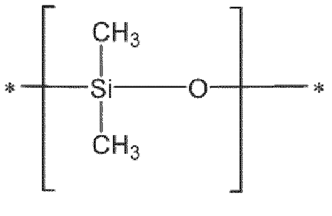
(Si-I)

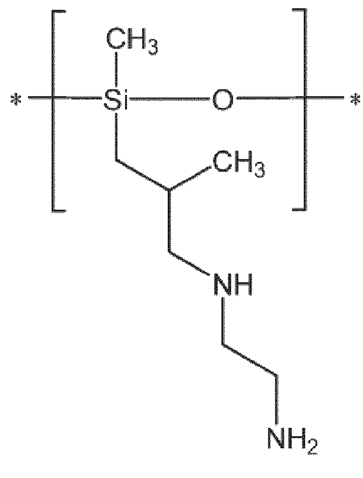
(Si-II), ,

and
(a2) at least one coloring compound, and
(a3) at least one preservative from the group of 2-phenoxyethanol, 4-hydroxybenzoic acid methyl ester, 4-hydroxybenzoic acid propyl ester, benzyl alcohol, 1-phenoxy-propan-2-ol, isopropanol, ethanol, zinc pyrithione,

benzoic acid, salicylic acid, sorbic acid, formic acid, propionic acid, 2-hydroxydiphenyl, 4-hydroxybenzoic acid, dehydroacetic acid, dibromohexamidine, 10-undecylenic acid, hexetidinum, trichlorocarban, triclosanum, 4-chloro-3,4-dimethylphenol, imidazolidinyl urea, hexamethylenetetramine, 1-(4-chlorophenoxy)-1-(imidazol-1-yl)-3,3-dimethyl-2-butanone, 1,3-bis-(hydroxymethyl)-5,5-dimethyl-2,4-imidazolidinedione, 1-hydroxy-4-methyl-6-(2,4,4-trimethylpentyl)-2-pyridone, bromochlorophene, 3-methyl-4-(1-methylethyl)phenol, 5-chloro-2-methyl-3(2H)-isothiazolone, 2-benzyl-4-chlorophenol, 2-chloroacetamide, chlorhexidine, 4,4-dimethyl-1,3-oxazolidine, hexamidinum, 5-ethyl-1-aza-3,7-dioxabicyclo-[3.3.0]-octane, chlorphenesin, sodium hydroxymethyl aminoacetate, benzyl hemiformal, 3-iodo-2-propynyl-butylcarbamate, methylisothiazolinone and ethyl lauroyl arginate, and
(a4) at least one non-ionic surfactant from the group of addition products of ethylene oxide to $C_8$-$C_{24}$ fatty alcohols.

2. The agent according to claim 1, **characterized in that** it contains, based on the total weight of the agent, one or more amino-functionalized silicone polymers (a1) in a total amount from 0.1 to 8.0 wt.%, preferably from 0.2 to 5.0 wt.%, more preferably from 0.3 to 3.0 wt.%, and very particularly preferably from 0.4 to 2.5 wt.%.

3. The agent according to one of claims 1 to 2, **characterized in that** it contains at least one coloring compound (a2) from the group of pigments, direct dyes, photochromic dyes and thermochromic dyes.

4. The agent according to one of claims 1 to 3, **characterized in that** it contains at least one coloring compound (a2) from the group of inorganic pigments, preferably selected from the group of colored metal oxides, metal hydroxides, metal oxide hydrates, silicates, metal sulfides, complex metal cyanides, metal sulphates, bronze pigments and/or from mica-based colored pigments which are coated with at least one metal oxide and/or a metal oxychloride.

5. The agent according to one of claims 1 to 4, **characterized in that** it contains at least one coloring compound (a2) from the group of organic pigments, preferably selected from the group of carmine, quinacridone, phthalocyanine, sorghum, blue pigments having the Color Index numbers CI 42090, CI 69800, CI 69825, CI 73000, CI 74100 or CI 74160, yellow pigments having the Color Index numbers CI 11680, CI 11710, CI 15985, CI 19140, CI 20040, CI 21100, CI 21108, CI 47000 or CI 47005, green pigments having the Color Index numbers CI 61565, CI 61570 or CI 74260, orange pigments having the Color Index numbers CI 11725, CI 15510, CI 45370 or CI 71105, and red pigments having the Color Index numbers CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 and/or CI 75470.

6. The agent according to one of claims 1 to 5, **characterized in that** it contains, based on the total weight of the agent (a), one or more pigments (a2) in a total amount from 0.01 to 10.0 wt.%, preferably from 0.1 to 5.0 wt.%, more preferably from 0.2 to 2.5 wt.%, and very particularly preferably from 0.25 to 1.5 wt.%.

7. The agent according to one of claims 1 to 6, **characterized in that** it contains (a3) 2-phenoxyethanol and/or 4-hydroxybenzoic acid methyl ester.

8. The agent according to one of claims 1 to 7, **characterized in that** it contains, based on the total weight of the agent, one or more preservatives (a3) in a total amount from 0.01 to 5.0 wt.%, preferably from 0.1 to 2.5 wt.%, more preferably from 0.15 to 1.0 wt.%, and very particularly preferably from 0.2 to 0.8 wt.%.

9. The agent according to one of claims 1 to 8, **characterized in that** it contains at least one non-ionic surfactant (a4) of formula (T-I),

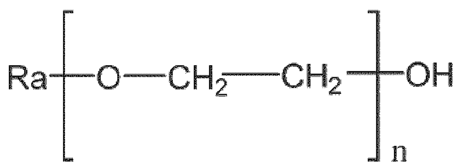

(T-I)

wherein

Ra represents a saturated or unsaturated, unbranched or branched $C_8$-$C_{24}$ alkyl group, preferably a saturated,

unbranched $C_{16}$ to $C_{18}$ alkyl group, and
N represents an integer from 80 to 120, preferably an integer from 90 to 110, and particularly preferably the integer 100.

**10.** The agent according to one of claims 1 to 9, **characterized in that** it contains at least one non-ionic surfactant (a4) of formula (T-II),

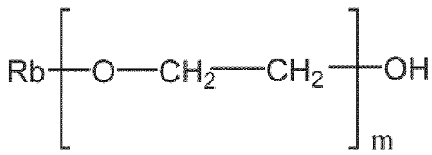

(T-II)

wherein

Rb represents a saturated or unsaturated, unbranched or branched $C_8$-$C_{24}$ alkyl group, preferably a saturated, unbranched $C_{16}$ to $C_{18}$ alkyl group, and
m represents an integer from 10 to 40, preferably an integer from 20 to 35, and particularly preferably the integer 30.

**11.** The agent according to one of claims 1 to 10, **characterized in that** it contains one or more fatty components from the group of $C_{12}$-$C_{30}$ fatty alcohols, $C_{12}$-$C_{30}$ fatty acid triglycerides, $C_{12}$-$C_{30}$ fatty acid monoglycerides, $C_{12}$-$C_{30}$ fatty acid diglycerides and/or hydrocarbons.

**12.** The agent according to one of claims 1 to 11, **characterized in that,** based on the total weight of the agent,

- the total content of all anionic surfactants contained in the agent is below 0.1 wt.%, and
- the total content of all cationic surfactants contained in the agent is below 0.1 wt.%.

**13.** The agent according to one of claims 1 to 12, **characterized in that,** based on the total weight of the agent,

- the total content of all anionic polymers contained in the agent is below 0.1 wt.%, and
- the total content of all cationic polymers contained in the agent is below 0.1 wt.%.

**14.** The agent according to one of claims 1 to 13, **characterized in that** it contains water and has a pH in the range of 7.0 to 11.5, preferably of 8.0 to 11.0, and particularly preferably of 8.5 to 10.5.

**15.** A method for dyeing keratinous material, in particular human hair, comprising the following steps:

(1) applying a dyeing agent according to one of claims 1 to 14 to the keratinous material,
(2) allowing the dyeing agent to act on the keratinous material, and
(3) rinsing out the dyeing agent using water.

**16.** The method according to claim 15, **characterized by**
(2) allowing the dyeing agent (a) to act on the keratinous material for a period of 30 seconds to 15 minutes, preferably 30 seconds to 10 minutes, and particularly preferably 1 to 5 minutes.

**Revendications**

**1.** Agent permettant la coloration d’une matière kératinique, en particulier de cheveux humains, contenant

(a1) au moins un polymère de silicone aminofonctionnalisé qui comprend des motifs structuraux de formule (Si-I) et de formule (Si-II)

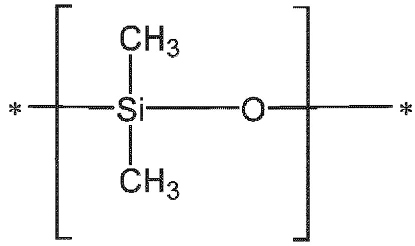

(Si-I)

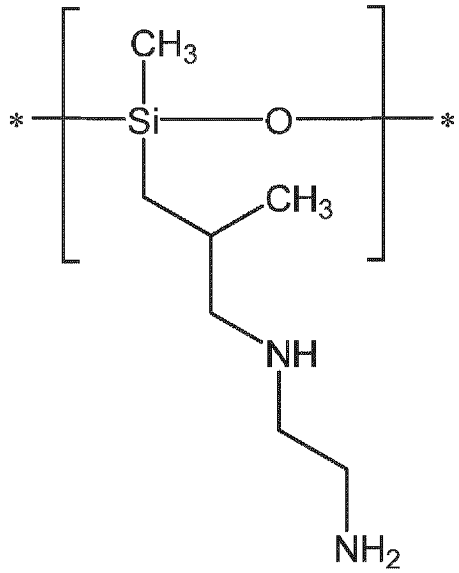

(Si-II),

et

(a2) au moins un composé colorant, et

(a3) au moins un conservateur choisi dans le groupe constitué de 2-phénoxyéthanol, ester méthylique d'acide 4-hydroxybenzoïque, ester propylique d'acide 4-hydroxybenzoïque, alcool benzylique, 1-phénoxy-propan-2-ol, isopropanol, éthanol, pyrithione de zinc, acide benzoïque, acide salicylique, acide sorbique, acide formique, acide propionique, 2-hydroxydiphényle, acide 4-hydroxybenzoïque, acide déshydracétique, dibromohexamidine, acide 10-undécylénique, hexétidine, trichlorocarbane, triclosanum, 4-chloro-3,4-diméthylphénol, imidazolidinylurée, hexaméthylènetétramine, 1-(4-chlorophénoxy)-1-(imidazol-1-yl)-3,3-diméthyl-2-butanone, 1,3-bis-(hydroxyméthyl)-5,5-diméthyl-2,4-imidazolidinedione, 1-hydroxy-4-méthyl-6-(2,4,4-triméthylpentyl)-2-pyridone, bromochlorophène, 3-méthyl-4-(1-méthyléthyl)phénol, 5-chloro-2-méthyl-3(2H)-isothiazolone, 2-benzyl-4-chlorophénol, 2-chloroacétamide, chlorhexidine, 4,4-diméthyl-1,3-oxazolidine, hexamidinum, 5-éthyl-1-aza-3,7-dioxabicyclo-[3.3.0]-octane, chlorphénésine, hydroxyméthylaminoacétate de sodium, benzylhémiformal, 3-iodo-2-propynylbutylcarbamate, méthylisothiazolinone et éthyl lauroyl arginate, et

(a4) au moins un tensioactif non ionique choisi dans le groupe des produits d'addition d'oxyde d'éthylène sur des alcools gras en $C_8$-$C_{24}$.

2. Agent selon la revendication 1, **caractérisé en ce qu'**il contient, par rapport au poids total de l'agent, un ou plusieurs polymères de silicone aminofonctionnalisés (a1) en une quantité totale allant de 0,1 à 8,0 % en poids, de préférence de 0,2 à 5,0 % en poids, plus préférablement de 0,3 à 3,0 % en poids et de manière tout particulièrement préférée de 0,4 à 2,5 % en poids.

3. Agent selon l'une des revendications 1 à 2, **caractérisé en ce qu'**il contient au moins un composé colorant (a2) choisi dans le groupe des pigments, colorants directs, colorants photochromiques et colorants thermochromiques.

4. Procédé selon l'une des revendications 1 à 3, **caractérisé en ce qu'**il contient au moins un composé colorant (a2) choisi dans le groupe des pigments inorganiques qui est de préférence choisi dans le groupe constitué d'oxydes métalliques colorés, hydroxydes métalliques colorés, oxydes métalliques hydratés colorés, silicates colorés, sulfures métalliques colorés, cyanures métalliques complexes colorés, sulfates métalliques colorés, pigments de bronze colorés et/ou de pigments colorés à base de mica recouverts d'au moins un oxyde métallique et/ou oxychlorure métallique.

5. Procédé selon l'une des revendications 1 à 4, **caractérisé en ce qu'**il contient au moins un composé colorant (a2) choisi dans le groupe des pigments organiques qui est de préférence choisi dans le groupe constitué de carmin, quinacridone, phtalocyanine, sorgho, pigments bleus comportant les numéros d'indice de couleur CI 42090, CI 69800, CI 69825, CI 73000, CI 74100, CI 74160, pigments jaunes comportant les numéros d'indice de couleur CI 11680, CI 11710, CI 15985, CI 19140, CI 20040, CI 21100, CI 21108, CI 47000, CI 47005, pigments verts comportant les numéros d'indice de couleur CI 61565, CI 61570, CI 74260, pigments orange comportant les numéros d'indice de couleur CI 11725, CI 15510, CI 45370, CI 71105, pigments rouges comportant les numéros d'indice de couleur CI

12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 et/ou CI 75470.

6. Agent selon l'une des revendications 1 à 5, **caractérisé en ce qu'il** contient, par rapport au poids total de l'agent (a), un ou plusieurs pigments (a2) en une quantité totale allant de 0,01 à 10,0 % en poids, de préférence de 0,1 à 5,0 % en poids, plus préférablement de 0,2 à 2,5 % en poids et de manière tout particulièrement préférée de 0,25 à 1,5 % en poids.

7. Agent selon l'une des revendications 1 à 6, **caractérisé en ce qu'il** contient (a3) du 2-phénoxyéthanol et/ou de l'ester méthylique d'acide 4-hydroxybenzoïque.

8. Agent selon l'une des revendications 1 à 7, **caractérisé en ce qu'il** contient, par rapport au poids total de l'agent, un ou plusieurs conservateurs (a3) en une quantité totale allant de 0,01 à 5,0 % en poids, de préférence de 0,1 à 2,5 % en poids, plus préférablement de 0,15 à 1,0 % en poids et de manière tout particulièrement préférée de 0,2 à 0,8 % en poids.

9. Agent selon l'une des revendications 1 à 8, **caractérisé en ce qu'il** contient au moins un tensioactif non ionique (a4) de formule (T-I),

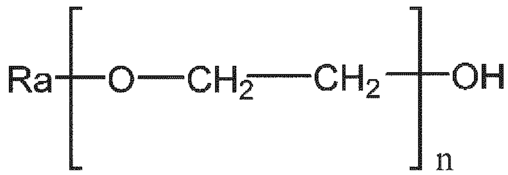

(T-I)

où

Ra représente un groupe alkyle en $C_8$-$C_{24}$ saturé ou insaturé, linéaire ou ramifié, de préférence un groupe alkyle en $C_{16}$-$C_{18}$ saturé linéaire, et
n représente un nombre entier allant de 80 à 120, de préférence un nombre entier allant de 90 à 110, et de manière particulièrement préférée le nombre 100.

10. Agent selon l'une des revendications 1 à 9, **caractérisé en ce qu'**il contient au moins un tensioactif non ionique (a4) de formule (T-II),

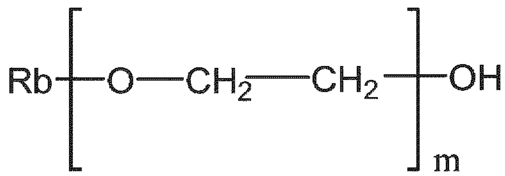

(T-II)

où

Rb représente un groupe alkyle en $C_8$-$C_{24}$ saturé ou insaturé, linéaire ou ramifié, de préférence un groupe alkyle en $C_{16}$-$C_{18}$ saturé linéaire, et
m représente un nombre entier allant de 10 à 40, de préférence un nombre entier allant de 20 à 35, et de manière particulièrement préférée le nombre 30.

11. Agent selon l'une des revendications 1 à 10, **caractérisé en ce qu'**il contient un ou plusieurs constituants gras choisi dans le groupe des alcools gras en $C_{12}$-$C_{30}$, triglycérides d'acides gras en $C_{12}$-$C_{30}$, monoglycérides d'acides gras en $C_{12}$-$C_{30}$, diglycérides d'acides gras en $C_{12}$-$C_{30}$ et/ou des hydrocarbures.

12. Agent selon l'une des revendications 1 à 11, **caractérisé en ce que,** par rapport au poids total de l'agent,

- la teneur totale en tous les tensioactifs anioniques contenus dans l'agent est inférieure à 0,1 % en poids, et

- la teneur totale en tous les tensioactifs cationiques contenus dans l'agent est inférieure à 0,1 % en poids.

**13.** Agent selon l'une des revendications 1 à 12, **caractérisé en ce que,** par rapport au poids total de l'agent,

- la teneur totale en tous les polymères anioniques contenus dans l'agent est inférieure à 0,1 % en poids, et
- la teneur totale en tous les polymères cationiques contenus dans l'agent est inférieure à 0,1 % en poids.

**14.** Agent selon l'une des revendications 1 à 13, **caractérisé en ce qu'**il contient de l'eau et possède un pH allant de 7,0 à 11,5, de préférence de 8,0 à 11,0 et de manière particulièrement préférée de 8,5 à 10,5.

**15.** Procédé permettant la coloration d'une matière kératinique, en particulier de cheveux humains, comprenant les étapes suivantes :

(1) application d'un agent de coloration selon l'une des revendications 1 à 14 sur la matière kératinique,
(2) action de l'agent de coloration sur la matière kératinique, et
(3) rinçage de l'agent de coloration avec de l'eau.

**16.** Procédé selon la revendication 15, **caractérisé par**
(2) l'action de l'agent de coloration (a) sur la matière kératinique pendant une durée allant de 30 secondes à 15 minutes, de préférence de 30 secondes à 10 minutes, et de manière particulièrement préférée de 1 à 5 minutes.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Nicht-Patentliteratur


- *CHEMICAL ABSTRACTS*, 106842-44-8
- *CHEMICAL ABSTRACTS*, 122-99-6
- *CHEMICAL ABSTRACTS*, 94-13-3
- *CHEMICAL ABSTRACTS*, 100-51-6
- *CHEMICAL ABSTRACTS*, 770-35-4
- *CHEMICAL ABSTRACTS*, 67-63-0
- *CHEMICAL ABSTRACTS*, 64-17-5
- *CHEMICAL ABSTRACTS*, 13463-41-7
- *CHEMICAL ABSTRACTS*, 65-85-0
- *CHEMICAL ABSTRACTS*, 69-72-7
- *CHEMICAL ABSTRACTS*, 110-44-1
- *CHEMICAL ABSTRACTS*, 64-18-6
- *CHEMICAL ABSTRACTS*, 79-09-4
- *CHEMICAL ABSTRACTS*, 90-43-7
- *CHEMICAL ABSTRACTS*, 99-96-7
- *CHEMICAL ABSTRACTS*, 520-45-6
- *CHEMICAL ABSTRACTS*, 93856-82-7
- *CHEMICAL ABSTRACTS*, 112-38-9
- *CHEMICAL ABSTRACTS*, 141-94-6
- *CHEMICAL ABSTRACTS*, 101-20-2
- *CHEMICAL ABSTRACTS*, 3380-34-5
- *CHEMICAL ABSTRACTS*, 88-04-0
- *CHEMICAL ABSTRACTS*, 39236- 46- 9
- *CHEMICAL ABSTRACTS*, 100-97-0
- *CHEMICAL ABSTRACTS*, 38083-17-9
- *CHEMICAL ABSTRACTS*, 6440-58-0
- *CHEMICAL ABSTRACTS*, 68890-66-4
- *CHEMICAL ABSTRACTS*, 15435-29-7
- *CHEMICAL ABSTRACTS*, 3228-02-2
- *CHEMICAL ABSTRACTS*, 26172-55-4
- *CHEMICAL ABSTRACTS*, 120-32-1
- *CHEMICAL ABSTRACTS*, 79-07-2
- *CHEMICAL ABSTRACTS*, 55-56-1
- *CHEMICAL ABSTRACTS*, 51200-87-4
- *CHEMICAL ABSTRACTS*, 3811-75-4
- *CHEMICAL ABSTRACTS*, 7747-35-5
- *CHEMICAL ABSTRACTS*, 886-74-8
- *CHEMICAL ABSTRACTS*, 70161-44-3
- *CHEMICAL ABSTRACTS*, 14548-60-8
- *CHEMICAL ABSTRACTS*, 55406-53-6
- *CHEMICAL ABSTRACTS*, 2682-20-4
- *CHEMICAL ABSTRACTS*, 60372-77-2
- *CHEMICAL ABSTRACTS*, 9005-00-9
- *CHEMICAL ABSTRACTS*, 68439-49-6